# EUROPEAN PATENT SPECIFICATION

(11) **EP 0 879 284 B1**
(45) Date of publication and mention of the grant of the patent: **29.07.2009**
(21) Application number: 97903998.9
(22) Date of filing: 28.01.1997
(51) Int. Cl.: C12N 15/00, C12N 15/63, C12N 15/79, C12N 15/09, A61K 48/00, A61K 31/39

(54) **GENE EXPRESSION VECTORS WHICH GENERATE AN ANTIGEN SPECIFIC IMMUNE RESPONSE AND METHODS OF USING THE SAME**
EXPRESSIONSVEKTOREN, DIE EINE ANTIGEN-SPEZIFISCHE IMMUNANTWORT INDUZIEREN, UND METHODEN FÜR IHRE VERWENDUNG.
VECTEURS D'EXPRESSION GENIQUE GENERANT UNE REPONSE IMMUNE SPECIFIQUE D'UN ANTIGENE ET LEURS PROCEDES D'UTILISATION

(30) Priority: 30.01.1996 US 593554
(43) Date of publication of application: 25.11.1998
(73) Proprietor: The Regents of The University of California, Oakland CA 94607 (US)
(72) Inventor: CARSON, Dennis, A., Del Mar, CA 92014 (US); RAZ, Eyal, San Diego, CA 92122 (US); ROMAN, Mark, San Diego, California 92126 (US)
(74) Representative: Brasnett, Adrian Hugh
(86) International application number: PCT/US1997/001277
(87) International publication number: WO 1997/028259

(56) References cited:
- EP-A- 0 468 520
- WO-A-96/02555
- RAZ E ET AL: "POTENTIAL ROLE OF IMMUNOSTIMULATORY DNA SEQUENCES (ISS) IN GENETIC IMMUNIZATION AND AUTOIMMUNITY" ARTHRITIS AND RHEUMATISM, LIPPINCOTT, PHILADELPHIA, US, vol. 39, no. 9, 1 September 1996 (1996-09-01), page 615 XP002058356 ISSN: 0004-3591
- YAMAMOTO S ET AL: "UNIQUE PALINDROMIC SEQUENCES IN SYNTHETIC OLIGONUCLEOTIDES ARE REQUIRED TO INDUCE INF AND AUGMENT INF-MEDIATED NATURAL KILLER ACTIVITY" JOURNAL OF IMMUNOLOGY, THE WILLIAMS AND WILKINS CO. BALTIMORE, US, vol. 148, no. 12, 15 June 1992 (1992-06-15), pages 4072-4076, XP002914069 ISSN: 0022-1767
- ZHAO Q ET AL: "EFFECT OF DIFFERENT CHEMICALLY MODIFIED OLIGODEOXYNUCLEOTIDES ON IMMUNE STIMULATION" BIOCHEMICAL PHARMACOLOGY, PERGAMON, OXFORD, GB, vol. 51, no. 2, 26 January 1996 (1996-01-26), pages 173-182, XP000610208 ISSN: 0006-2952
- ROMAN MARK ET AL: "Immunostimulatory DNA sequences function as T helper-1-promoting adjuvants" NATURE MEDICINE, NATURE PUBLISHING, CO, US, vol. 3, no. 8, August 1997 (1997-08), pages 849-854, XP002188113 ISSN: 1078-8956
- KEKKAKU, 1994, Vol. 69, No. 9, YAMAMOTO S., "Mode of Action of Oligonucleotide Fraction Extracted from Mycobacterium Bovis BCG", pages 571-574.
- BIOCHEMICAL AND BIOPHYSICAL RESEARCH COMMUNICATIONS, 30 September 1993, Vol. 195, No. 3, CARLBERG C., "RXR-Independent Action of the Receptors for Thyroid Hormone Retinoid Acid and Vitamin D on Inverted Palindromes", pages 1345-1353.
- J. OF IMMUNOLOGY, 15 June 1992, Vol. 148, No. 12, YAMAMOTO et al., "Unique Palindromic Sequences in Synthetic Oligonucleotides Are Required to Induce IFN and Augment INF-Mediated Natural Killer Activity", pages 4072-4076.
- GENE, 14 May 1990, Vol. 89, No 2, OHI et al., "Construction and Replication of an Adeno-Associated Virus Expression Vector that Contains Human beta-Globin cDNA", pages 279-282.
- MICROBIOL. IMMUNOL., 1994, Vol. 38, No. 10, YAMAMOTO et al., "Lipofection of Synthetic Oligodeoxyribonucleotide Having a Palindromic Sequence of AACGTT to Murine Splenocytes Enhances Interferon Production and Natural Killer Activity", pages 831-836.
- ANTISENSE RESEARCH AND DEVELOPMENT, 1994, Vol. 4, YAMAMOTO et al., "Ability of Oligonucleotides with Certain Palindromes to Induce Interferon Production and Augment Natural Killer Cell Activity is Associated with Their Base Length", pages 119-122.

## Description

### Field of the Invention

The invention relates to methods and reagents for immunizing a host against an antigen. Specifically, the invention relates to recombinant expression vectors for use as an adjuvant for vaccination of a host against an antigen and methods for using such vectors.

### Background of the Invention

Immunization of a host against an antigen has traditionally been accomplished by repeatedly vaccinating the host with an immunogenic form of the target antigen. An emerging area of vaccine design involves the use of cytokines to direct and boost immune responses to a target antigen (which may lower the total dose of tolerizing antigen required to induce protection).

For example, the IL-12 cytokine is believed to encourage proliferation of CD4⁺ TH1 cells (active in cell-mediated immunity) and cytotoxic T lymphocytes (CTLs) in preference to TH2 cells (active in humoral immunity). IL-12 has also been shown to substantially augment proliferation and differentiation of lymphocytes, including cytotoxic T lymphocytes. There is evidence that IL-12 plays a critical role in confering immune protection against intracellular antigens (see, e.g., Scott, J.Immunol., 147:3149 (1991) [protective effect against *L.major* in mice in the presence of IL-12 lost when source of IL-12 eliminated]). However, administration of purified cytokines to a host risks toxicity, particularly at dosages sufficient to stimulate the host immune system. The same risk is posed by administration of target antigen to the host in a conventional vaccination scheme.

For these reasons, gene transfer (for introduction of a protein antigen and/or cytokine into a host by administration of a gene which encodes the antigen and/or cytokine of interest) is an intriguing alternative to traditional, antigen-based immunization protocols. However, the viral vectors commonly used for *in situ* gene expression may integrate endogenous genetic material into the host's genome and presence potential health risks associated with damage to the genetic material in host cells.

Recently, "naked" gene expression vectors (e.g., plasmids for expression of a target polypeptide) have been shown to express encoded polypeptides in vivo. One of the earliest steps in this field was taken in 1984 at the NIH. Seeger, *et al*. reported data which indicated that intrahepatic injection of naked, cloned plasmid DNA for squirrel hepatitis into squirrels produced both viral infection and the formation of antiviral antibodies in the squirrels (Seeger, et al., Proc.Nat'l.Acad.Sci USA, 81:5849-5852, 1984). Several years later, Felgner, *et al*., reported that they obtained expression of viral protein from plasmids injected into the skeletal muscle tissue of mice (Felgner, et al., Science, 247:1465, 1990; see also, PCT application WO 90/11092).

More recently, research concerning potential therapeutic uses for naked gene expression vectors has focused on enhancing gene expression through use of different promotors, delivery vehicles and routes of administration (see, e.g., Stribling, et al., Proc. Natl. Acad. Sci. USA, 89:11277-11281, 1992 [expression following aerosol delivery of a gene occurred with use of a liposomal delivery system]; and, Tang, et al., Nature, 356:152-154, 1992 [injection with a vaccine "gun" of an hGH plasmid coupled to colloidal gold beads]).

However, use of muscle as a route for gene vaccine administration has certain drawbacks. For example, researchers working with the.University of Ottawa recently observed that "[s]triated muscle is the only tissue found to be capable of taking up and expressing reporter genes that are transferred in the form of plasmid DNA...but our findings indicate that fibers damaged by the injection procedure do not take up and express plasmid DNA." (Davis, et al., Human Gene Therapy, 4:151-159, 1993).

The production of humoral immune responses to the expression products of naked gene vectors in tissues other than muscle has sparked interest in the use of the vectors as vehicles for vaccines and to deliver immunostimulatory cytokines to target cells (e.g., in recent human trials, IL-2 and IL-4 were delivered by retroviral vectors and ex vivo transformed cells). However, an obstacle to the use of naked gene expression vectors for vaccination has been the relatively rarity of cellular immune responses to expressed antigen.

In general, a cellular immune response to antigen (particularly through expansion of the cytotoxic T cell population) can be expected to be necessary to long-term protection against the antigen. However, any somatic cell that expresses antigen must first release the antigen into the extracellular space for uptake by antigen presenting cells before a class I restricted cytotoxic T cell response can to the antigen can be induced (see, e.g., Huang, et al., Science, 264:961-965, 1994). Thus, it appears that enhancement of gene expression without stimulation of antigen presenting cell activity and induction of a cellular immune response will be insufficient to allow successful use of naked gene expression vectors in vaccination protocols.

WO96/02555, published on 1 February 1996, relates to certain oligonucleotides containing unmethylated cytosine-guanine dinucleotides. These oligonucleotides were used to activate B lymphocytes.

### Summary of the Invention

In one aspect, the invention relates recombinant expression vectors for use in naked gene immunization ("naked gene expression vectors"). The naked gene expression vectors of the invention include immunostimulatory polynucleotides which elicit a vigorous cell-mediated immune response. The invention also includes naked gene expression vectors for use in manipulating cellular immune responses toward the TH1 compartment.

In particular, the invention provides use of an immunostimulatory polynucleotide comprising the sequence 5'-cytosine-guanine-3', in the preparation of a medicament wherein the medicament further comprises an antigen, for use in the treatment by administration to the mucosa of a condition requiring stimulation of a Th1 lymphocyte response to said antigen in a mammal, wherein the condition is an infectious disease.

The invention further provides a composition comprising an antigen and an immunostimulatory polynucleotide comprising the sequence 5'-cytosine-guanine-3', for use in a treatment of a condition requiring stimulation of a Th1 lymphocyte response to said antigen wherein the condition is an infectious disease in a mammal and wherein said composition is administered to the mucosa.

As used with respect to the invention, the term "naked gene expression vector" refers to plasmids or cosmids which include at least one non-coding, immunostimulatory polynucleotide region, preferably also encode a peptide of interest (e.g., antigens and cytokines) and are not associated with a delivery vehicle (e.g., liposomes, colloidal particles and the like). One of the principal advantages touted for non-viral vectors has been the lack of immune responses stimulated by the vector itself. However, the inventors have discovered that vector-mediated stimulation of the host immune system is a desirable goal, and may be necessary, to permit use of naked gene expression vectors as efficient vaccination vehicles.

In particular, the design of the inventive gene expression vectors exploits the discovery that enhancement of antigen expression by recombinant expression vectors is not sufficient to provoke a protective immune response against the expressed antigen. Specifically, the relatively high expression levels achieved from those non-viral vectors commonly tested for use in gene immunization (which lack the immunostimulatory polynucleotides of the invention) may provoke humoral immune responses of varying intensity, but rarely produce the cell-mediated immune responses necessary for long-term protection against antigen.

To the latter end, the naked gene expression vectors for use in the invention include immunologically active regions of nucleic acids which are believed to selectively stimulate in vivo transcription of interferon α (IFNα) by antigen presenting cells (APCs), which in turn stimulates production of IL-12 and proliferation of cytotoxic T lymphocytes (CTLs) . According to the method of the invention, antigen uptake by APCs is augmented and the host's cell-mediated response to antigen is enhanced, thus boosting the host's cellular immunity against the antigen. In this respect, the naked gene expression vectors of the invention are particularly useful for immunizing a host against intracellular (e.g., viral) infection. The vectors are also of particular use in stimulating the TH1 compartment in preference to the TH2 compartment, thus suppressing IgE production in response to expressed antigen.

The naked gene expression vectors of for use in the invention include one or more non-coding, immunostimulatory polynucleotides which include at least one dinucleotide sequence consisting of adjacent, unmethylated cytosine-guanine (CG) nucleotides. Immunostimulatory polynucleotides useful in the invention may be double or single-stranded DNA or RNA, but will preferably form double-stranded palindromes. Most preferably, each CG dinucleotide sequence of the immunostimulatory polynucleotides of the invention will be flanked on one side (upstream or downstream) by two or more purine nucleotides and on the other side (upstream or downstream) by two or more pyrimidine nucleotides. The naked gene expression vectors may also encode polypeptides of interest, such as antigens and cytokines.

Given the immunostimulatory properties of the immunostimulatory polynucleotides of for use in the invention, their inclusion in other recombinant gene expression vectors and antigen-based vaccine compositions can also be expected to enhance the anti-antigen immune response of the host. Thus, another aspect of the invention includes viral recombinant gene expression vectors and non-viral recombinant gene expression vectors associated with delivery vehicles (e.g., liposomes or colloidal particles) into which immunostimulatory polynucleotides of the invention have been inserted. In addition, using the same techniques by which immunostimulatory polynucleotides of for use in the invention are incorporated into viral gene expression vectors, the polynucleotides may be incorporated into live viral vaccines to augment the immune response to viral antigens.

In another aspect, the invention comprises related to a method for immunizing a host against antigen using the naked gene expression vectors of the invention. According to a preferred method of the invention, naked gene expression vectors are introduced into tissues of the host having a relatively high concentration of antigen presenting cells (APCS) therein (e.g., skin or mucosa) as compared to other host tissues (e.g., muscle). Introduction of the naked gene expression vectors into the host may be by any suitable means, but will preferably be made by relatively non-invasive means such as chemical or mechanical irritation of the epidermis or upper cellular layers of mucosa. With co-administration of antigen or a recombinant expression vector encoding antigen, the naked gene expression vectors of the invention serve as adjuvants to enhance the immune response of a host to the antigen.

Although the invention is not to be limited by any particular mechanism of action, it is expected that introduction of the naked gene expression vectors of the invention into host APCs will encourage APC presentation of antigen by Class I processing pathways for stimulation of TH1 immune responses in preference to TH2 immune responses. A further advantage provided by the method use of the invention whereby antigen is encoded by recombinant expression vectors injected into skin or mucosa is that protective immune responses may be provoked by relatively low doses of antigen (e.g., about 50 µg or less). Therefore, although IL-12 is believed to suppress cellular protein expression (and could therefore shut down antigen presentation over time), sufficient antigen expression can be achieved in the invention to provide the host immunity sought.

### Brief Description of the Drawings

FIGURE 1 is a map of a pCMV-LacZ vector which contains two copies of the immunostimulatory polynucleotide palindrome AACGTT (SEQ.ID.No.1).
FIGURE 2 is a graph comparing the anti-β-galactosidase antibody response of mice immunized intradermally with either the pCMV-LacZ naked gene expression vector, a vector which lacks an immunostimulatory polynucleotide (pKCB-LacZ), or combinations of the latter vector with KanR (KCB) and AmpR (ACB) genes or pCMV-GMCSF (encoding granulocyte-monocyte colony stimulating factor).
FIGURE 3a is a map of a pKCB-LacZ vector which lacks an immunostimulatory polynucleotide; FIGURE 3b is a map of a pKCB-LACZ vector into which one copy of the polynucleotide of SEQ.ID.No.1 was inserted (pKCB-laaZ); FIGURE 3c is a map of a pKCB-LacZ vector into which two copies of the polynucleotide of SEQ.ID.No.1 were inserted (pKCB-2aaZ).
FIGURE 4 is a graph comparing the anti-β-galactosidase IgG antibody response of mice immunized intradermally with respectively, the pCMV-LacZ vector, the pKCB-LacZ vector, the pKCB-laaZ vector, the pKCB-2aaZ vector, and combinations of the pKCB-LacZ vector with KanR (KCB) and AmpR (ACB) genes.
FIGURE 5 is a graph comparing the IgG antibody response of mice to β-galactosidase after intradermal immunization with, respectively, pCMV-LacZ or the pKCB-LacZ vector alone and in combination with pUC-19.
FIGURE 6 is a graph depicting the cellular immune (CTL) response of mice after immunization with, respectively, the pKCB-LacZ vector, the pCMV-LacZ vector or a control vector.
FIGURE 7 is a graph depicting the cellular immune (CTL) response of mice after immunization with, respectively, the pKCB-LacZ vector or the same vector in combination with the pUC-19 vector.
FIGURE 8 is a map of a pVDREtk vector suitable for insertion of immunostimulatory polynucleotides of the invention, which vector contains a ligand-inducible nuclear receptor promoter.
FIGURE 9a represents the anti-viral antigen antibody responses of mice immunized intradermally with a pCMV-NP (viral nucleoprotein) vector; FIGURE 9b compares the responses of mice injected intramuscularly with the same vector.
FIGURE 10 depicts the level of LacZ gene expression detected in Chinese hamster ovary (CHO) cells transformed with either the pCMV-LacZ or pKCB-LacZ plasmids.
FIGURE 11 is a Kaplan-Meyer survival curve for mice vaccinated against a viral antigen according to the method of the invention and for unvaccinated mice.
FIGURE 12 is a graph depicting the memory T cell responses to antigen in mice immunized with pCMV-LacZ intradermally or intramuscularly.
FIGURE 13 is a graph depicting the IgG 2a responses to antigen of mice immunized intradermally with pCMV-LacZ, intramuscularly with pCMV-LacZ or antigen.
FIGURE 14 is a graph depicting the IgG 2a responses to antigen of mice immunized with intradermally with pCMV-LacZ, intramuscularly with pCMV-LacZ or antigen.
FIGURE 15 is a graph depicting the IgG 2a response of the mice described with respect to FIGURE 13 after boosting.
FIGURE 16 is a graph depicting the IgG 1 response of the mice decribed with respect.to FIGURE 13 after boosting.
FIGURE 17 is a graph of the anti-β-galactosidase IgE antibody responses of mice immunized with the pCMV-LacZ plasmid.
FIGURE 18 depicts the anti-NP (influenza nucleoprotein) responses of mice immunized by absorption of a pCMV-NP vector or antigen through skin treated with a keratinolytic agent.
FIGURE 19 depicts the relative levels of IFN-γ production by splenocytes from mice immunized with pKCB-LacZ, pCMV-LacZ or a combination dose of pKCB-LacZ and pUC-19, then challenged with antigen.
FIGURE 20 depicts the relative levels of IL-4 production by splenocytes from mice immunized with pKCB-LacZ, pCMY-Lac2 or a combination dose of pKCB-LacZ and pUC-19, then challenged with antigen.

### Description of the Preferred Embodiments

### I. Immunostimulatory Polynucleotides for Use in the Gene Expression Vectors of the Invention

### A. Non-coding, immunostimulatory polynucleotide sequences

The non-coding immunostimulatory polynucleotides of used in the invention are those which stimulate CTLS activity (as compared to responses to control vectors having no immunostimulatory polynucleotide of the invention) and, preferably, stimulate production of interferons (INF) by lymphocytes. In double-stranded form, such polynucleotides include at least one palindromic region (i.e., a region where the nucleotide sequence of one strand is the reverse complement of a corresponding region of the complementary strand). Each palindromic region may be as little as about 6 nucleotides in length (and of any maximum length), excluding complementary strand sequences, extrapalindromic regions, inserted restriction sites and linkers.

Further, each palindromic region of the immunostimulatory polynucleotides of used in the invention includes an unmethylated CG dinucleotide sequence; i.e., at least two adjacent nucleotides, where one such nucleotide is a cytosine and the other such nucleotide is a guanine. In double-stranded molecules, each CG dinucleotide sequence present in the palindromic region of the immunostimulatory polynucleotide is palindromic; i.e., the cytosine of the CG sequence on one strand is paired with a guanine in a CG sequence on the complementary strand. In single-stranded molecules, the relative position of each CG sequence in the immunostimulatory polynucleotide is preferably 5'-CG-3' (i.e., the C is in the 5' position with respect to the G in the 3' position).

Most preferably, each CG dinucleotide sequence of each immunostimulatory polynucleotide of the invention is flanked by at least two purine nucleotides (e.g., GA or AA) and at least two pyrimidine nucleotides (e.g., TC or TT) to enhance the B lymphocyte stimulatory activity of the immunostimulatory polynucleotide (see, e.g., Krieg, et al., Nature, 374:546-549, 1995).

The immunostimulatory polynucleotides of used in the invention are inserted into a naked gene expression vector by techniques well known to those of ordinary skill in the art (see, e.g., Section II, *infra*). Suitable polynucleotide sequences for use as restriction sites, linkers and the like may be included in the immunostimulatory polynucleotide of the invention to be inserted into the naked gene expression vector. The immunoatimulatory polynucleotides of used in the invention may be inserted at any location in the naked gene expression vector and will preferably be inserted at least twice so the resulting vector contains at least two palindromic regions according to the invention.

Exemplary immunostimulatory polynucleotides of the invention include:
Single-stranded DNA: AACGTT (SEQ.ID.No.1)
Double-stranded (palindromic) DNA: AACGTT (SEQ.ID.No.2) TTGCAA

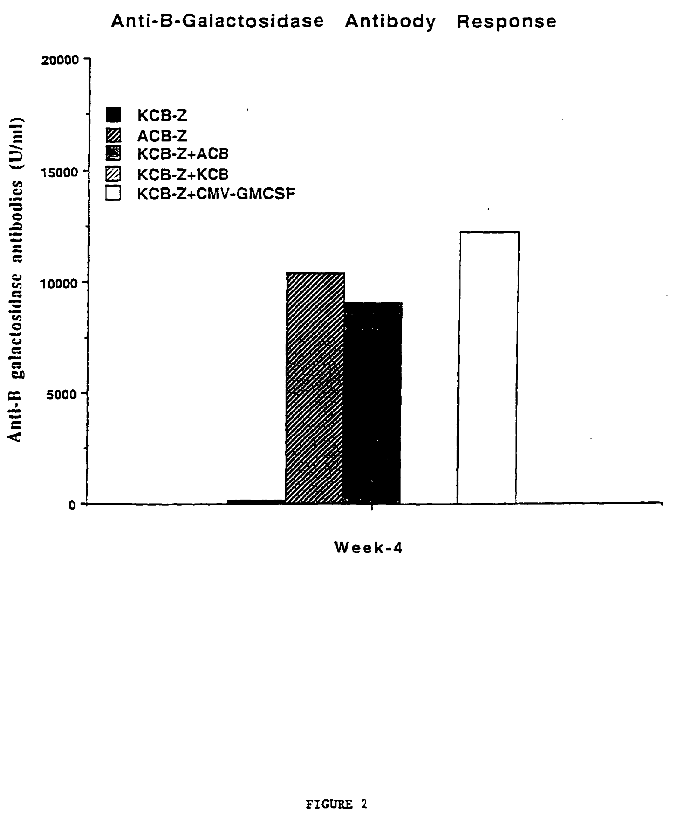

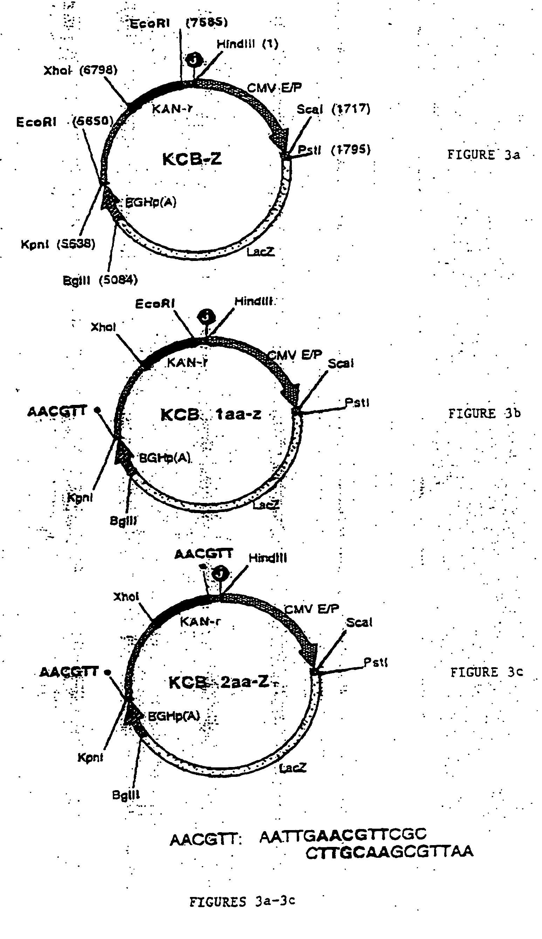

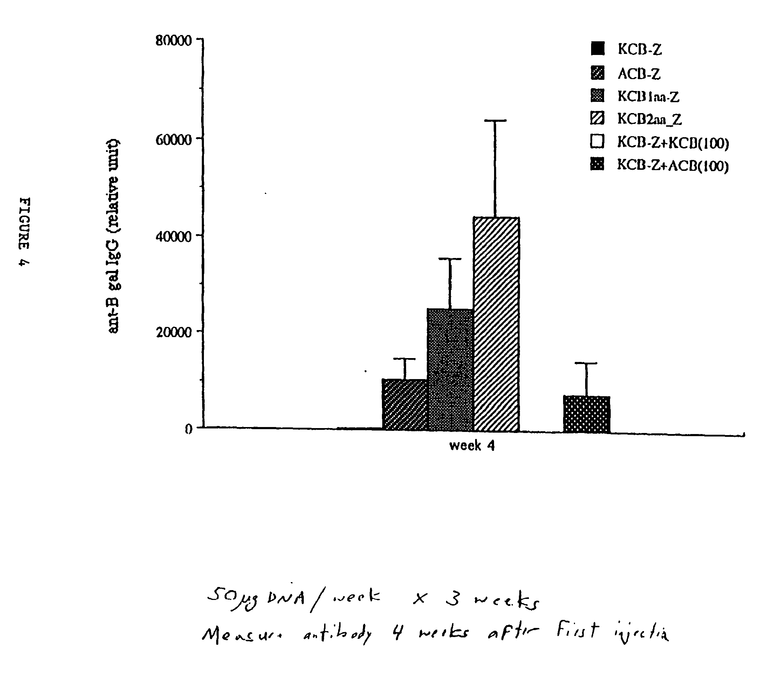

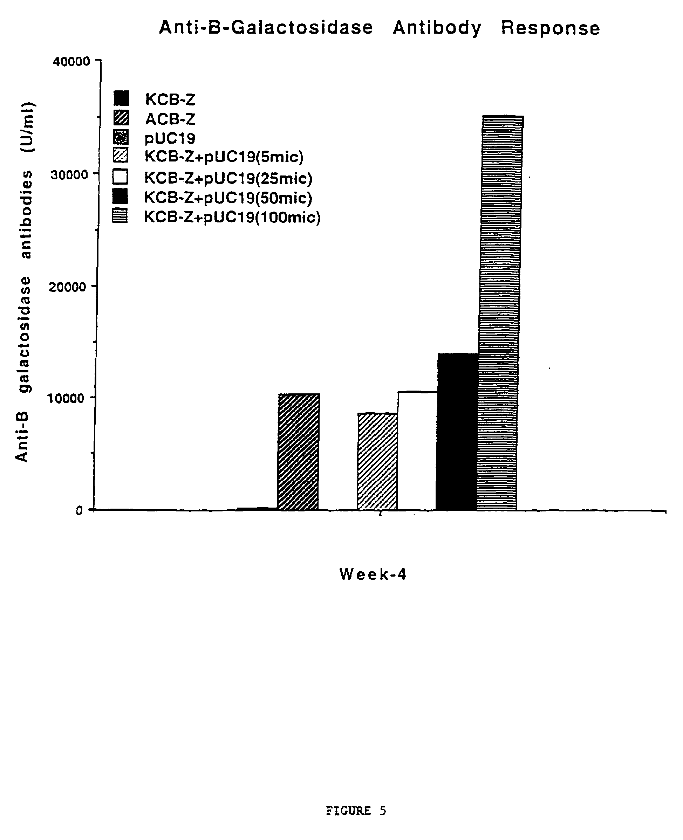

If an immunostimulatory polynucleotide such as the one described above is absent from a recombinant gene expression vector, little humoral or cellular immune response to an expressed antigen is stimulated even where levels of antigen expression is increased. For example, as shown in FIGURE 2, intradermal injection of mice with a plasmid (pCMV-LacZ; which includes two copies of the immunostimulatory polynucleotide of SEQ.ID.No.1) stimulated a substantially greater anti-antigen antibody response to the encoded reporter molecule (β-galactosidase, or "LacZ") than was stimulated in response to intradermal injection of a plasmid containing a kanamycin resistance enzyme encoding gene (KCB) which lacks an immunostimulatory polynucleotide of the invention (see, the vector map for pKCB-LacZ in FIGURE 3a; and Example II). Immunostimularity was conferred on the pKCB-LacZ vector when one or more copies of the immunostimulatory polynucleotide of SEQ.ID.No. 1 were inserted into the vector (to form pKCB-1aaZ and pKCB-2aaZ; see, vector maps at FIGURE 3b and FIGURE 3c; data shown in FIGURE 4; and Example II), as well as after co-administration of a separate plasmid which contains two copies of the immunostimulatory polynucleotide of SEQ.ID.No.1 (pUC-19) or vector encoding granulocyte stimulating factor (pCMV-GMCSF) (FIGURE 5).

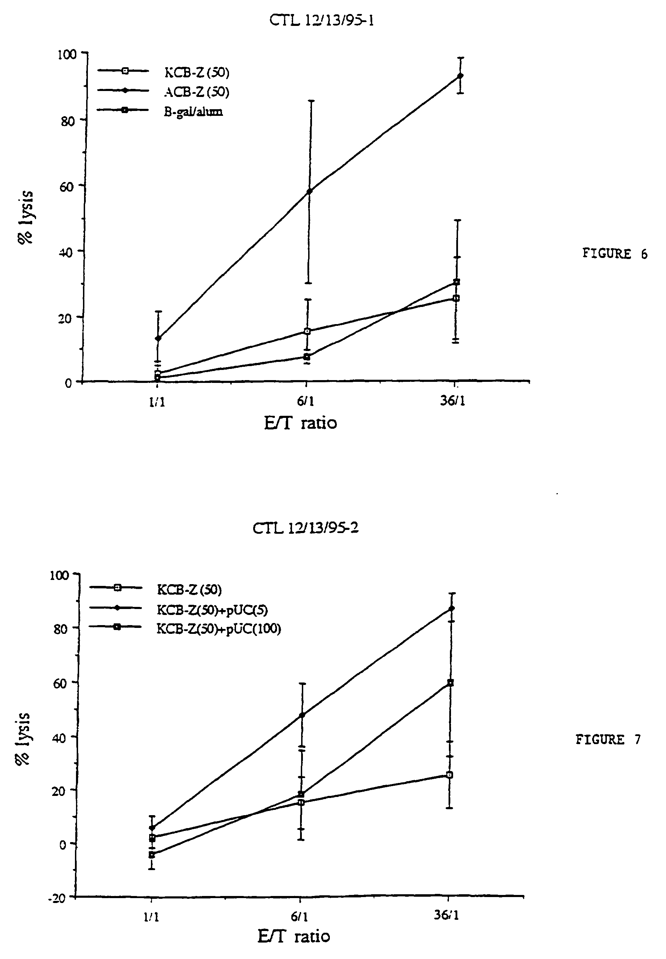

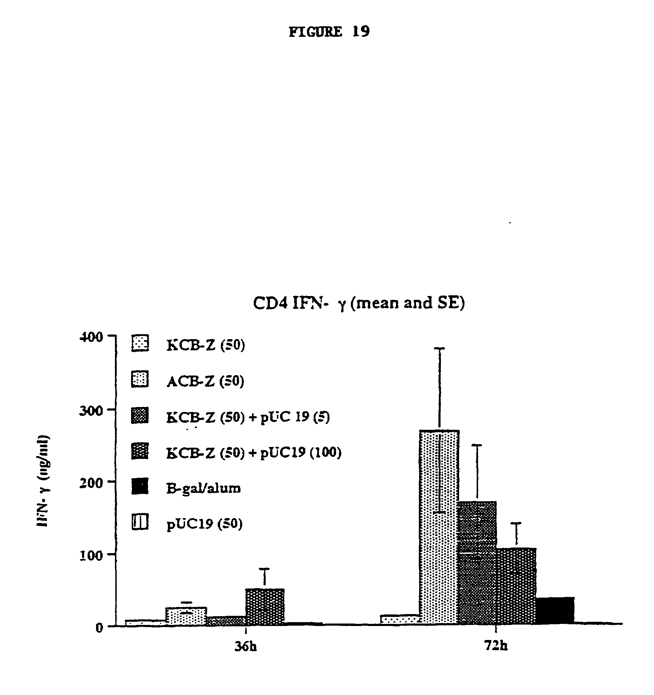

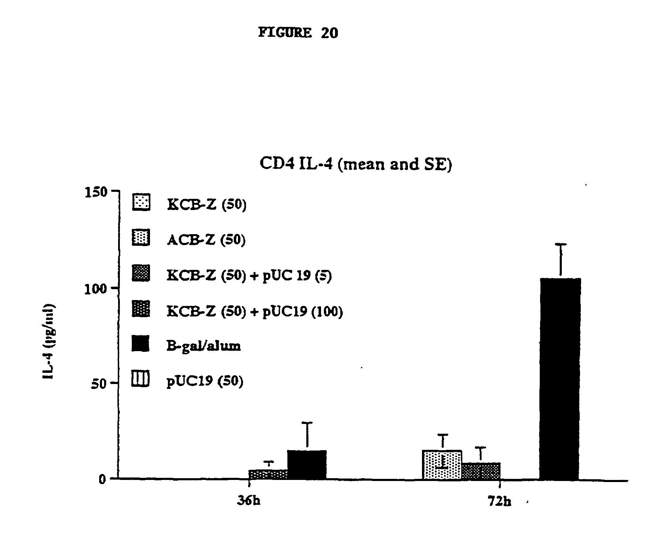

Similarly, the cellular immune responses of such mice to the pCMV-LacZ plasmid and to co-administration of pUC-19 with a KCB plasmid were substantially greater than the response of mice injected intradermally with the pKCB-LacZ plasmid which lacks an immunostimulatory polynucleotide of the invention (see, FIGURE 6 [CTL lysis of cells transfected with pKCB-LacZ, pCMV-LacZ or control]; FIGURE 7 [CTL lysis of cells transfected with pKCB-LacZ or pKCB-LacZ with different quantities of pUC-19]; Example III; FIGURE 19 [IFN-γ production by spleen cells from mice immunized with pKCB-LacZ (low production levels), pCMV-LacZ (higher production levels) or a combination of pKCB-LacZ and pUC-19 (higher production levels)]; FIGURE 20 (IL-4 production by the same spleen cells tested for IFN-γ production in FIGURE 19]; and Example IX).

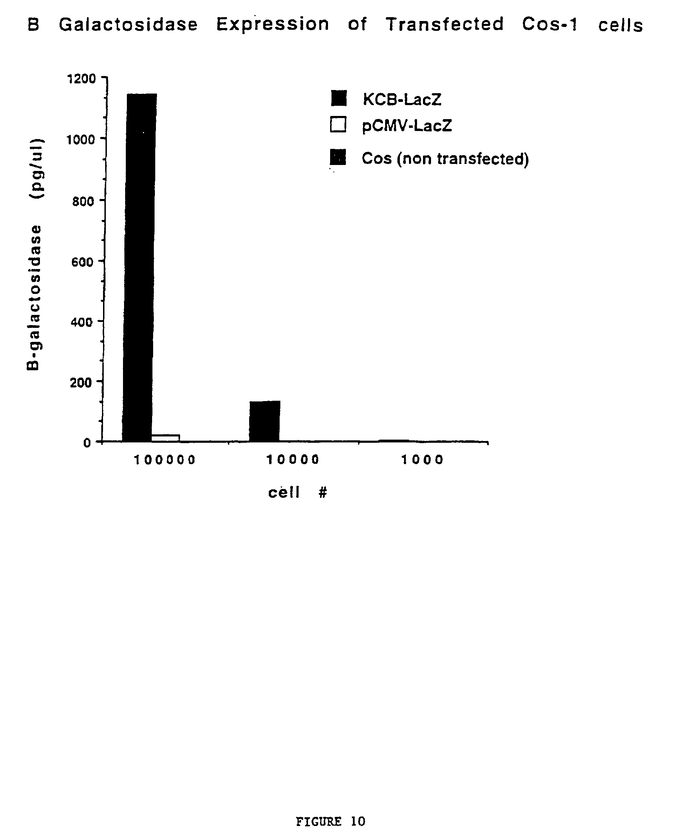

The lack of an immune response after injection of the unmodified pKCB plasmids (as compared to plasmids including the immunostimulatory polynucleotide of SEQ.ID.No.1) was particularly surprising in view of the greater levels of antigen expression obtained *in vivo* after injection of the pKCB-LacZ plasmid (as compared to the pCMV-LacZ plasmid) (see, FIGURE 10). Logically, one would expect greater expression of antigen to be reflected in the magnitude of immune response to the antigen. Yet, absent an immunostimulatory polynucleotide in a non-coding region of the expression vector, this expectation is not fulfilled in vivo.

Thus, contrary to present theory in the art, increasing levels of antigen expression will not necessarily enhance the immune response of an animal to the expressed antigen. In the context of the invention, it is the immunostimulatory polynucleotides of the invention, rather than just the magnitude of antigen expression, which enhance host immune responses to expressed antigen in gene immunization protocols. This activity on the part of the immunostimulatory polynucleotides of the invention (and recombinant gene expression vectors which contain them), as well as the beneficial adjuvant effect of that activity, is unexpected given the general view in the art that DNA is a poor immunogen and that immune responses to gene expression vectors for use in gene replacement and vaccination protocols should be avoided (as compared to the desired anti-antigen response sought in the latter context).

Other exemplary immunostimulatory polynucleotides of the invention include (only one strand of each palindrome is shown):

| | |
|---|---|
| GCGCGC (SEQ.ID.No.3) | GACGTC (SEQ.ID.No.4) |
| AGCGCT (SEQ.ID.No.5) | ATCGAT (SEQ.ID.No.6) |
| CGATCG (SEQ.ID.No.7) | CGTACG (SEQ.ID.No.8) |
| CGCGCG (SEQ.ID.No.9) | TCGCGA (SEQ.ID.No.10) |
| ACCGGT (SEQ.ID.No.11) | ACGT (SEQ.ID.No.12) |
| GACGATCGTC (SEQ.ID.No.13) | ACGATCGT (SEQ.ID.No.14) |
| CGACGATCGTCG (SEQ.ID.No.15) | |
| CGACGACGATCGTCGTCG (SEQ.ID.No.16) | |
| CAACGTTG (SEQ.ID.No.17) | ACAACGTTGT (SEQ.ID.No.18) |
| AACAACGTTGTT (SEQ.ID.No.19) | |
| CAACAACGTTGTTG (SEQ.ID.No.20) | |

Those of ordinary skill in the art will readily be able to identify other palindromic polynucleotides which (a) possess the structural characteristics of the immunostimulatory polynucleotides of the invention described above; and, (b) stimulate both humoral and cellular immune responses *in vivo* as measured by conventional detection techniques (such as those described in the Examples, *infra*). As incorporated into naked gene expression vectors, all such polynucleotides are within the scope of this invention.

### B. Preparation of immunostimulatory, antigenic and cytokine-encoding polynucleotides for insertion into the naked gene expression vectors of the invention.

As used herein, "polynucleotide" refers to a polymer of deoxyribonucleotides or ribonucleotides, in the form of a separate fragment or as a component of a larger construct. The non-coding, immunostimulatory polynucleotides of the invention may be double or single-stranded DNA or RNA inserted into recombinant expression vectors, preferably naked gene expression vectors. Such polynucleotides must also be either non-replicating or engineered by means well known in the art so as not to replicate into the host genome. The recombinant gene expression vectors of the invention may also include coding regions for expression of antigens, cytokines, T cell epitopes and other immunotherapeutically significant polypeptides.

Screening procedures which rely on nucleic acid hybridization make it possible to isolate any polynucleotide sequence from any organism, provided the appropriate probe or antibody is available. Oligonucleotide probes, which correspond to a part of the sequence encoding the protein in question, can be synthesized chemically. This requires that short, oligopeptide stretches of amino acid sequence must be known. The DNA sequence encoding the protein can also be deduced from the genetic code, however, the degeneracy of the code must be taken into account.

For example, a cDNA library believed to contain a polynucleotide of interest can be screened by injecting various mRNA derived from cDNAs into oocytes, allowing sufficient time for expression of the cDNA gene products to occur, and testing for the presence of the desired cDNA expression product, for example, by using antibody specific for a peptide encoded by the polynucleotide of interest or by using probes for the repeat motifs and a tissue expression pattern characteristic of a peptide encoded by the polynucelotide of interest. Alternatively, a cDNA library can be screened indirectly for expression of peptides of interest having at least one epitope using antibodies specific for the peptides. Such antibodies can be either polyclonally or monoclonally derived and used to detect expression product indicative of the presence of cDNA of interest.

Polynucleotides for use in the invention can also be synthesized using techniques and nucleic acid synthesis equipment which are well-known in the art. For reference in this regard, see Ausubel, et al., Current Protocols in Molecular Biology, Chs. 2 and 4 (Wiley Interscience, 1989) (genomic DNA); and, Maniatis, et al., Molecular Cloning: A Laboratory Manual (Cold Spring Harbor Lab., New York, 1982) (cDNA). For ease of construction and use, synthesized polynucleotides and cDNAs are generally preferred for use in the recombinant gene expression vectors of the invention.

In addition to the immunostimulatory polynucleotides of for use in the invention, the recombinant gene expression vectors of the invention may be constructed to include coding regions for peptides of therapeutic or immunostimulatory interest. For example, a mixture of polynucleotides or separately coadministered group of polynucleotides may be of use in immunizing a host against more than one antigen and/or to further stimulate a host immune response (by, for example, including a gene operatively encoding for an immunosuppressive cytokine such as TGFβ or a relevant histo-compatibility protein in the recombinant gene expression vector).

The recombinant gene expression vectors of for use in the invention may also encode peptides having more than one biological activity. For example, a polynucleotide operatively encoding for an immunostimulatory peptide may be coupled to or administered with a polynucleotide operatively encoding an antibody in such a way that both peptide and antibody will be expressed. To illustrate, administration of genes which will jointly express IL-2 and anti-gp71 may (based on results obtained with the IL-2 protein) result in localization of the antibody in tumor tissue developed in response to murine leukemia virus (MuLV) in mice (see, re results obtained with concurrent administration of IL-2/anti-gp71 mAb's, Schultz, et al., Cancer Res., 50:5421-5425, 1990). Further, the same vector may also encode an antigen, T cell epitope, cytokine or other polypeptides in combination.

Up to 200 polynucleotide sequences under the control of a single promoter can be expressed by an appropriate plasmid or cosmid. Such "cocktail" vectors will be of particular use in treating infections by agents of different species which cause similar symptoms. For example, there are over 100 known species of rhinoviruses which cause respiratory illnesses having similar clinical symptoms. Rather than undertaking the identification of the particular infecting species (a laborious and often inexact process), a cocktail vaccine could be administered according to the method of the invention which is capable of stimulating an immune response to many different rhinoviruses. This approach also allows for the construction of a vaccine to various strains of HIV, using pooled isolates of envelope genes from different patients (which genes may, if necessary, then be amplified).

Known polynucleotide sequences for genes encoding such polypeptides of interest will be readily accessible to, or known by, those of ordinary skill in the art.

### II. Methods for Construction of Reconbiannt and Naked Gene Expression Vectors

The recombinant gene expression vectors of for use in the invention are preferably plasmids or cosmids which include immunostimulatory polynucleotides of the invention, but may also be viruses or retroviruses. As discussed above, the vectors may also include gene(s) which operatively encode a peptide of interest (e.g., antigens and cytokines). Most preferably, the vectors are "naked"; i.e., not associated with a delivery vehicle (e.g., liposomes, colloidal particles and the like). For convenience, the term "plasmid" as used in this disclosure will refer to plasmids or cosmids, depending on which is appropriate to use for expression of the peptide of interest (where the choice between the two is dictated by the size of the gene encoding the peptide of interest). "Operatively encode" refers to a gene which is associated with all of the regulatory sequences required for expression of a polypeptide.

Immunostimulatory polynucleotides of for use in the invention, as well as polynucleotides which encode antigens or cytokines, may be conjugated to or used in association with other polynucleotides that operatively code for regulatory proteins that control the expression of these polypeptides or may contain recognition, promoter and secretion sequences. Those of ordinary skill in the art will be able to select regulatory polynucleotides and incorporate them into the recombinant gene expression vectors of the invention (if not already present therein) without undue experimentation. For example, suitable promoters for use in murine or human systems and their use are described in Ausubel, Current Protocols in Molecular Biology, supra at Ch. 1.

In general, plasmid vectors which may be used in the invention contain promoters and control sequences which are derived from species compatible with the host cell. For example, *E. coli* is typically transformed using pBR322, a plasmid derived from an E. coli species (Bolivar, et al., Gene, 2:95, 1977). pBR322 contains genes for ampicillin (AMPR) and tetracycline resistance (the former of which includes polynucleotide fragments useful in the invention) and thus provides easy means for identifying transformed cells. However, for use in humans, the U.S. Food and Drug Administration presently prohibits use of recombinant expression vectors which may confer ampicillin resistance to the host. The pBR322 plasmid, or other microbial plasmid must also contain or be modified to contain promoters and other control elements commonly used in recombinant DNA construction.

"Control sequence(s)" or "control region" refers to specific sequences at the 5' and 3' ends of eukaryotic genes which may be involved in the control of either transcription or translation. Virtually all eukaryotic genes have an AT-rich region located approximately 25 to 30 bases upstream from the site where transcription is initiated. Another sequence found 70 to 80 bases upstream from the start of transcription of many genes is a CCAAT region where X may be any nucleotide. At the 3'end of most eukaryotic genes is an AATAAA sequence which may be the signal for additional of the poly A tail to the 3'end of the transcribed mRNA.

For those vectors for use in recombinant gene expression vectors of the invention that include genes which operatively encode polypeptides of interest, preferred promoters controlling transcription from vectors in mammalian host cells may be obtained from various sources, for example, the genomes of viruses such as polyoma, Simian Virus 40 (SV40), adenovirus, retroviruses, hepatitis-B virus and most preferably cytomegalovirus, or from heterologous mammalian promoters, e.g. beta actin promoter. The early and later promoters of the SV40 virus are conveniently obtained as an SV40 restriction fragment which also contains the SV40 viral origin of replication (Fiers, et al, Nature, 273:113, 1978). The immediate early promoter of the human cytomegalovirus is conveniently obtained as a HindIII E restriction fragment (Greenaway, et al., Gene, 18:355-360, 1982). Promoters from the host cell or related species also are useful herein.

Promoters suitable for use with prokaryotic hosts illustratively include the β-lactamase and lactose promoter systems (Chang, et al., Nature, 275:615, 1978; and Goeddel, et al., Nature, 281:544, 1979), alkaline phosphatase, the tryptophan (trp) promoter system (Goeddel, Nucleic Acids Res., 8:4057, 1980) and hybrid promoters such as the taq promoter (de Boer, et al., Proc. Natl. Acad. Sci. USA, 80:21-25, 1983). However, other functional bacterial promoters are suitable. Their nucleotide sequences are generally known in the art, thereby enabling a skilled worker to ligate them to a polynucleotide which encodes the peptide of interest (Siebenlist, et al., Cell, 20:269, 1980) using linkers or adapters to supply any required restriction sites.

In addition to prokaryotes, eukaryotic microbes such as yeast cultures may also be used as source for control sequences. *Saccharomyces cerevisiae,* or common baker's yeast is the most commonly used eukaryotic microorganism in this context, although a number of other strains are commonly available.

Suitable promoting sequences for use with yeast hosts include the promoters for 3-phosphoglycerate kinase (Hitzeman, et al., J. Biol. Chem., 255:2073, 1980) or other glycolytic enzymes (Hess, et al. J. Adv. Enzyme Reg. 7:149, 1968; and Holland, Biochemistry, 17:4900, 1978) such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degraded enzymes associated with nitrogen metabolism, metallothionine, glyceraldehyde-3-phosphate dehydrogenase, and enzymes responsible for maltose and galactose utilization. Yeast enhancers also are advantageously used with yeast promoters.

Transcription of DNA encoding a polypeptide of interest by higher eukaryotes is increased by inserting an enhancer sequence into the vector. Enhancers are cis-acting elements of DNA, usually about from 10-300 bp, that act on a promoter to increase its transcription. Enhancers are relatively orientation and position independent having been found 5' (Laimins, et al., Proc.Natl.Sci.Acad.USA, 78:993, 1981) and 3' (Lusky, et al., Mol. Cell Bio., 3:1108, 1983) to the transcription unit, and within an intron (Banerji, et al., Cell, 33:729, 1983) as well as within the coding sequence itself (Osborne, et al., Mol. Cell Bio., 4:1293 1984). Many enhancer sequences are now known from mammalian genes (globin, elastase, albumin, α-feto-protein and insulin). Typically, however, an enhancer from a eukaryotic cell virus will be used. Examples include the SV40 enhancer on the late side of the replication origin (bp 100-270), the cytomegalovirus early promoter enhancer, the polyoma enhancer on the late side of the replication origin, and adenovirus enhancers.

Expression vectors that contain a gene which operatively encodes a polypeptide and are intended to be introduced into eukaryotic host cells (yeast, fungi, insect, plant, animal, human or nucleated cells from other multicellular organisms) will also contain sequences necessary for the termination of transcription which may affect mRNA expression. Expression vectors may also contain a selection gene, also termed a selectable marker. Examples of suitable selectable markers for mammalian cells which are known in the art include dihydrofolate reductase (DHFR), thymidine kinase or neomycin. When such selectable markers are successfully transferred into a mammalian host cell, the transformed mammalian host cell can survive if placed under selective pressure (i.e., by being conferred with drug resistance or genes altering the nutrient requirements of the host cell).

Those of ordinary skill in the art will be familiar with, or may readily ascertain the identity of, viruses and retroviruses for use as recombinant expression vectors having the non-coding, immunostimulatory polynucleotides of the invention. Such artisans will also be able to construct non-viral vectors associated with delivery vehicles such as liposomes or colloidal particles without undue experimentation. Therefore, only a brief summary regarding such viral and non-viral vectors will be provided here for review.

For those embodiments of the invention which do not rely on APC recognition of polynucleotides as antigen, a colloidal dispersion system may be used for targeted delivery. Colloidal dispersion systems include macromolecule complexes, nanocapsules, microspheres, beads, and lipid-based systems including oil-in-water emulsions, micelles, mixed micelles, and liposomes. The preferred colloidal system of this invention is a liposome.

Liposomes are artificial membrane vesicles which are useful as delivery vehicles *in vitro* and *in vivo.* It has been shown that large unilamellar vesicles (LUV), which range in size from 0.2-4.0 µm can encapsulate a substantial percentage of an aqueous buffer containing large macromolecules. RNA, DNA and intact virions can be encapsulated within the aqueous interior and be delivered to cells in a biologically active form (Fraley, *et al*., *Trends Biochem. Sci.,* 6:77, 1981). In addition to mammalian cells, liposomes have been used for delivery of polynucleotides in plant, yeast and bacterial cells. In order for a liposome to be an efficient gene transfer vehicle, the following characteristics should be present: (1) encapsulation of the genes encoding the antisense polynucleotides at high efficiency while not compromising their biological activity; (2) preferential and substantial binding to a target cell in comparison to non-target cells; (3) delivery of the aqueous contents of the vesicle to the target cell cytoplasm at high efficiency; and (4) accurate and effective expression of genetic information (Mannino, et al., Biotechniques, 6:682, 1988).

The composition of the liposome is usually a combination of phospholipids, particularly high-phase-transition-temperature phospholipids, usually in combination with steroids, especially cholesterol. Other phospholipids or other lipids may also be used. The physical characteristics of liposomes depend on pH, ionic strength, and the presence of divalent cations.

Examples of lipids useful in liposome production include phosphatidyl compounds, such as phosphatidylglycerol, phosphatidylcholine, phosphatidylserine, phosphatidylethanolamine, sphingolipids, cerebrosides, and gangliosides. Particularly useful are diacylphosphatidylglycerols, where the lipid moiety contains from 14-18 carbon atoms, particularly from 16-18 carbon atoms, and is saturated. Illustrative phospholipids include egg phosphatidylcholine, dipalmitoylphosphatidylcholine and distearoylphosphatidylcholine.

The targeting of liposomes can be classified based on anatomical and mechanistic factors. Anatomical classification is based on the level of selectivity, for example, organ-specific, cell-specific, and organelle-specific. Mechanistic targeting can be distinguished based upon whether it is passive or active. Passive targeting utilizes the natural tendency of liposomes to distribute to cells of the reticulo-endothelial system (RES) in organs which contain sinusoidal capillaries. Active targeting, on the other hand, involves alteration of the liposome by coupling the liposome to a specific ligand such as a monoclonal antibody, sugar, glycolipid, or protein, or by changing the composition or size of the liposome in order to achieve targeting to organs and cell types other than the naturally occurring sites of localization.

The surface of the targeted delivery system may be modified in a variety of ways. In the case of a liposomal targeted delivery system, lipid groups can be incorporated into the lipid bilayer of the liposome in order to maintain the targeting ligand in stable association with the liposomal bilayer. Various linking groups can be used for joining the lipid chains to the targeting ligand.

Various viral vectors that can be utilized in the invention include adenovirus, herpes virus, vaccinia, or, preferably, an RNA virus such as a retrovirus. Preferably, the retroviral vector is a derivative of a murine or avian retrovirus. Examples of retroviral vectors in which a single foreign gene can be inserted include, but are not limited to: Moloney murine leukemia virus (MoMuLV), Harvey murine sarcoma virus (HaMuSV), murine mammary tumor virus (MuMTV), and Rous Sarcoma Virus (RSV). A number of additional retroviral vectors can incorporate multiple genes. All of these vectors can transfer or incorporate a gene for a selectable marker so that transduced cells can be identified and generated.

By inserting one or more sequences of interest into the viral vector, along with another gene which encodes the ligand for a receptor on a specific target cell, for example, the vector is now target specific. Retroviral vectors can be made target specific by inserting, for example, a polynucleotide encoding a sugar, a glycolipid, or a protein. Preferred targeting is accomplished by using an antibody to target the retroviral vector. Those of skill in the art will know of, or can readily ascertain without undue experimentation, specific polynucleotide sequences which can be inserted into the retroviral genome to allow target specific delivery of the retroviral vector containing the polynucleotides of interest. A separate vector can be utilized for targeted delivery of a replacement gene to the cell(s), if needed. In antisense therapy, an antisense oligonucleotide and the replacement gene may also be delivered via the same vector since the antisense oligonucleotide is specific only for target gene containing a polymorphism.

Since recombinant retroviruses are defective, they require assistance in order to produce infectious vector particles. This assistance can be provided, for example, by using helper cell lines that contain plasmids encoding all of the structural genes of the retrovirus under the control of regulatory sequences within the LTR. These plasmids are missing a nucleotide sequence that enables the packaging mechanism to recognize an RNA transcript for encapsidation. Helper cell lines that have deletions of the packaging signal include, but are not limited to, ψ2, PA317 and PA12, for example. These cell lines produce empty virions, since no genome is packaged. If a retroviral vector is introduced into such helper cells in which the packaging signal is intact, but the structural genes are replaced by other genes of interest, the vector can be packaged and vector virion can be produced.

It will be appreciated that the same techniques which are utilized to incorporate the immunostimulatory polynucleotides of the invention into viral gene expression vectors may be used to incorporate the sequences into live and attenuated live viruses for use as vaccines. Such modified viral vaccines can be expected to have greater immunostimulatory properties than would be found in the viral vaccine itself.

Construction of suitable vectors containing desired coding, non-coding and control sequences employ standard ligation techniques. Isolated plasmids or DNA fragments are cleaved, tailored, and relegated in the form desired to construct the plasmids required.

For example, for analysis to confirm correct sequences in plasmids constructed, the ligation mixtures may be used to transform a host cell and successful transformants selected by antibiotic resistance where appropriate. Plasmids from the transformants are prepared, analyzed by restriction and/or sequenced by, for example, the method of Messing, et al., (Nucleic Acids Res., 9:309, 1981), the method of Maxam, et al., (Methods in Enzymology, 65:499, 1980), or other suitable methods which will be known to those skilled in the art. Size separation of cleaved fragments is performed using conventional gel electrophoresis as described, for example, by Maniatis, et al., (Molecular Cloning, pp. 133-134, 1982).

Host cells may be transformed with the expression vectors of this invention and cultured in conventional nutrient media modified as is appropriate for inducing promoters, selecting transformants or amplifying genes. The culture conditions, such as temperature, pH and the like, are those previously used with the host cell selected for expression, and will be apparent to the ordinarily skilled artisan.

For purposes of monitoring expression, recombinant gene expression vectors may be modified to include genes which operatively encode known reporter polypeptides. For example, the pRSV lac-Z DNA vector described in Norton, et al., Mol. Cell. Biol., 5:281, (1985), may produce β-galactosidase with protein expression. Luciferase and chloramphenicol acetyl transferase ("CAT"; see, e.g., Gorman, *et al*., supra, re construction of a pRSV-CAT plasmid) may also be used. Convenient plasmid propogation may be obtained in E. coli (see, e.g., Molecular Cloning: *A Laboratory Manual,* supra.)

Two particularly preferred plasmid vectors for modification and use according to the invention are the pRSV (Rous sarcoma virus) and pCMV (cytomegalovirus) promoter vectors. Of these promoters, CMV is preferred for polynucleotides to be introduced into tissue other than muscle. This preference is based on observations that higher levels of expression are achieved in this context when the CMV promoter is employed.

A suitable protocol for isolation of the RSV promotor and its use in construction of a plasmid vector is described in Gorman, et al., Proc. Natl. Acad. Sci, USA, 79:6777, (1982). Other preferred plasmid vectors are pREP7 and pREV which are commercially available from Invitrogen of San Diego, California. For cloning of polynucleotides, a particularly suitable plasmid for production of mRNA is the pSP64T cloning vector described by Kreig, et al., Nucleic Acids Res., 12:7057-7070, (1984). Any cDNA containing an initiation codon can be introduced into this plasmid and mRNA prepared from the expressed DNA templates using conventional techniques.

Also, particularly useful vector constructs for use according to the invention are those which contain a promoter that can be switched "on" or "off" after the vector has been administered to a patient such as the ligand-inducible nuclear receptor promoters. Recombinant gene expression vectors containing such promoters are of particular use in vaccination protocols wherein the vector is introducted into the skin or mucosa, where expression can be controlled by applying the inducing ligand for absorption into the site at which the vector has been introduced.

Nuclear receptors represent a family of transcriptional enhancer factors that act by binding to specific DNA sequences found in target promoters known as response elements. Specific members of the nuclear receptor family include the primary intracellular targets for small lipid-soluble ligands, such as vitamin D₃ and retinoids, as well as steroid and thyroid hormones ("activating ligands").

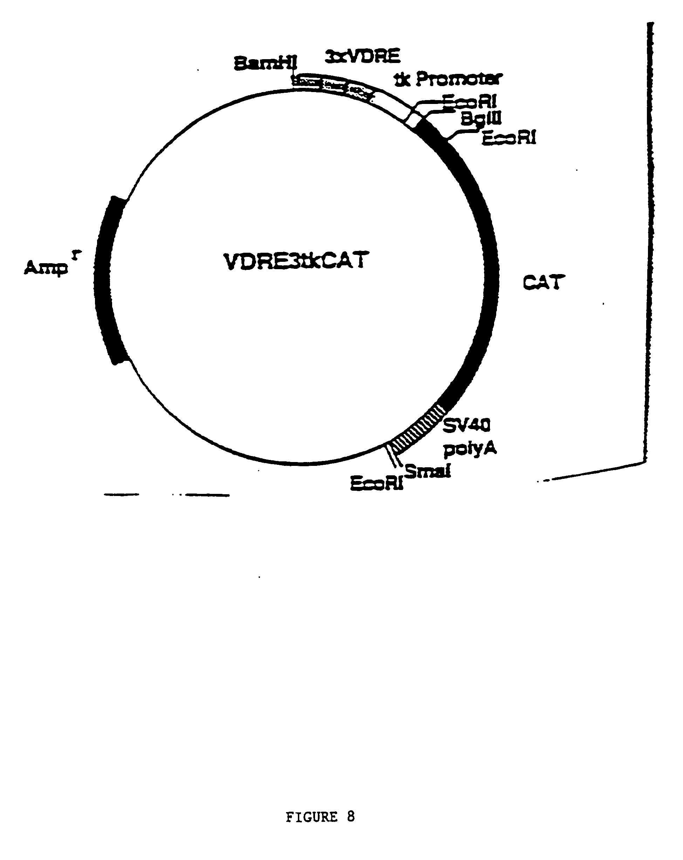

Nuclear receptors activated by specific activating ligands are well suited for use as promoters in eukaryotic expression vectors since expression of genes can be regulated simply by controlling the concentration of ligand available to the receptor. For example, glucocorticoid-inducible promoters such as that of the long terminal repeat of the mouse mammary tumor virus (MMTV) have been widely used in this regard because the glucocorticoid response elements are expressed in a wide variety of cell types. One expression system which exploits glucocorticoid response elements responsive to a wide variety of steroid hormones (e.g., dexamethasone and progesterone) is a pGREtk plasmid (containing one or more rat tyrosine amino transferase glucocorticoid response elements upstream of the herpes simplex virus thymidine kinase (tk) promoter in pBLCATB+), transfected in HeLa cells (see, Mader and White, Proc.Nacl.Acad.Sci USA, 90:5603-5607, 1993 [pGRE2tk]; and, Klein-Hitpass, et al., Cell, 46:1053-1061, 1986 [pBLCAT8+]; the disclosures of which are incorporated herein by this reference to illustrate knowledge in the art concerning construction of suitable promoters derived from nuclear receptor response elements ("NRRE promoters")). The pGREtk promoter (see, map at FIGURE 8) is particularly effective in stimulating controlled overexpression of cloned genes in eukaryotic cells (Mader and White, *supra* at 5607).

Another particularly suitable NRRE promoter for use in the invention is one which is inducible by the vitamin D₃ compound 1,25-dihydroxyvitamin D₃ and non- hypercalcemic analogs thereof (collectively, "vitamin D₃ activating ligands"). NRRE promoters inducible by vitamin D₃ activating ligands contain the vitamin D₃ receptor (VDR) response elements PurG(G/T)TCA which recognizes direct repeats separated by 3 base pairs. Vitamin D₃ response elements are found upstream of human osteocalcin and mouse osteopontin genes; transcription of these genes is activated on binding of the VDR *(see, e.g.,* Morrison and Eisman, J.Bone Miner.Res., 6:893-899, 1991; and, Ferrara, et al., J.Biol.Chem., 269:2971-2981, 1994, the disclosures of which are incorporated herein by this reference to illustrate knowledge in the art of vitamin D₃ responsive inducible promoters). Recent experimental results from testing of a recombinant expression vector containing the mouse osteopontin VDR upstream of a truncated herpes simplex virus thymidine kinase (tk) promoter suggested that 9-cis-retinoic acid can augment the response of VDR to 1,25-hydroxyvitamin D₃ (see, Carlberg, et al., Nature, 361:657-660,1993).

Ferrara, *et al.* also described vitamin D₃ inducible promoters in recombinant expression vectors constructed using multiple copies of a strong VDR; in particular, the mouse osteopontin VDR (composed of a direct repeat of PurGTTCA motifs separated by 3 base pairs). This VDR conforms to the PurGG/TTCA consensus motifs which have previously been shown to be responsive not only to vitamin D₃, but also to thyroid hormone and/or retinoic acid. As many as three copies of the mouse VDR was inserted into pBLCAT8+; immediately upstream of the herpes simplex virus tk promoter (see, e.g., FIGURE 8 [map of pVDREtk]). Transfection of the resulting VDREtk vector into COS cells (producing a "VDR expression system") proved to be particularly useful in that COS cells contain the nuclear retinoid X receptor (RXR) that has been shown to act as an auxiliary factor for binding of VDR to its response element.

The VDR expression system (and functionally equivalent expression systems under the control of, for example, human osteocalcin gene promoter) is uniquely suited for use in the invention. Specifically, expression of a polynucleotide administered to a mammal according to the invention by epidermal or dermal routes (particularly the former) in a vitamin D₃ responsive expression system can be switched on by topical administration of a 1,25-dihydroxyvitamin D₃ preparation at the point of entry (and off by withdrawing the vitamin D₃ preparation and/or modulated by applying or withdrawing a source of retinoic acid to or from the point of entry). Conveniently, 1,25-dihydroxyvitamin D₃ and nonhypercalcemic analogs thereof have been approved for use in topical preparations by the United States Food and Drug Administration for the treatment of psoriasis and are commercially available.

In vivo tests of the NRRE promoters indicate that they are inducible on systemic exposure to their corresponding response elements. Given the expected retention of polynucleotides administered dermally or epidermally at the point of entry (thus making them available for exposure to topically absorbed response elements), it can be reasonably predicted that use of NRRE promoters for expression of such polynucleotides will also permit their *in vivo* control through topical administration of appropriate NRRE promoter activating ligands (e.g., 1,25-dihydroxyvitamin D₃ transcriptional activators with a VDR expression vector for expression of the polynucleotide of interest).

Thus, use of an NRRE promoter recombinant gene expression vector for administration and expression of coding and immunostimulatory non-coding polynucleotides according to the invention permits control of expression to, for example, switch on expression when dosing is needed or switch off expression in the event of an adverse reaction to the expressed protein or peptide.

### III. Pharmaceutical Preparations of Recombinant Gene Expression Vectors

Compositions of recombinant gene expression vectors may be placed into a pharmaceutically acceptable suspension, solution or emulsion. Suitable mediums include saline and may, for indications which do not rely on antigen presenting cells for delivery of the polynucleotides into target tissue, liposomal preparations. However, as discussed further infra with respect to the method of the invention, it is preferred that the recombinant gene expression vectors of the invention not be conjugated to a liposome or used with any other material which may impede recognition of the vector as foreign by the host immune system.

More specifically, pharmaceutically acceptable carriers preferred for use with the naked gene expression vectors of the invention may include sterile aqueous of non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/ aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers (such as those based on Ringer's dextrose), and the like. Preservatives and other additives may also be present such as, for example, antimicrobials, antioxidants, chelating agents, and inert gases and the like. Further, a composition of recombinant gene expression vectors may be lyophilized using means well known in the art, for subsequent reconstitution and use according to the invention.

Isotonic buffered solution is the preferred medium for maximal uptake of the naked gene expression vectors. Further, use of absorption promoters, detergents, chemical irritants or mechanical irritation means is also preferred to enhance transmission of recombinant gene expression vector compositions through the point of entry. For reference concerning general principles regarding promoters and detergents which have been used with success in mucosal delivery of organic and peptide-based drugs, see Chien, *Novel Drug Delivery Systems,* Ch. 4 (Marcel Dekker, 1992). Specific information concerning known means and principles of nasal drug delivery are discussed in Chien, supra at Ch 5. Examples of suitable nasal absorption promoters are set forth at Ch. 5, Tables 2 and 3; milder agents are preferred. Further, known means and principles of transdermal drug delivery are also discussed in Chien, supra, at Ch. 7. Suitable agents for use in the method of this invention for mucosal/nasal delivery are also described in Chang, et al., Nasal Drug Delivery, "Treatise on Controlled Drug Delivery", Ch. 9 and Table 3-4B thereof, (Marcel Dekker, 1992). Suitable agents which are known to enhance absorption of drugs through skin are described in Sloan, Use of Solubility Parameters from Regular Solution Theory to Describe Partitioning-Driven Processes, Ch. 5, "Prodrugs: Topical and Ocular Drug Delivery" (Marcel Dekker, 1992), and at places elsewhere in the text.

It is expected that these techniques (and others which are conventionally used to facilitate drug delivery) may be adapted to preparation of recombinant gene expression vector for use in the methods of the invention by those of ordinary skill in the art without undue experimentation. In particular, although the approaches discussed in the preceding paragraphs have not, to the inventors' knowledge, been previously used for polynucleotide delivery, it is believed that they are suitable for use to that end. For that reason, the references identified above, while not essential to the inventive compositions and methods, are incorporated herein by this reference.

### IV. Methods for In Vivo Use of the Naked Gene Expression Vectors of the Invention

### A. Definitions

The following definitions will be of use in understanding the method of the invention.
a. "Antigen Presenting Cells", or "APC's" include known APC's such as Langerhans cells, veiled cells of afferent lymphatics, dendritic cells and interdigitating cells of lymphoid organs. The definition also includes mononuclear cells such as (1) lymphocytes and macrophages which take up and express polynucleotides according to the invention in skin and (2) mononuclear cells depicted on histological photographs contained herein. These cells are not tissue cells but are likely to be antigen presenting cells. The most important of these with respect to the present invention are those APC's which are known to be present in high numbers in epithelia and thymus dependent areas of the lymphoid tissues, including epidermis and the squamous mucosal epithelia of the buccal mucosa, vagina, cervix and esophagus (areas with "relatively high" concentrations of APC's). In addition to their definitions set forth below, therefore, "skin" and "mucosa" as used herein particularly refer to these sites of concentration of APC's. Further, "processional APCs" shall refer to cells whose primary purpose is antigen presentation; i.e., bone marrow derived cells.
b. "Detergrents/Absorption Promoters" refers to chemical agents which are presently known in the art to facilitate absorption and transfection of certain small molecules, as well as peptides.
c. "Iontophoresis" refers to a known means of transdermal transmission presently used to deliver peptides continuously to a host. More specifically, it is a process that facilitates the transport of ionic species by the application of a physiologically acceptable electrical current. This process and other transdermal transmission means are described in Chien, et al. Transdermal Drug Delivery, "Novel Drug Delivery Systems", Ch. 7, part C, (Marcel Dekker, 1992), the relevant disclosures of which are incorporated herein by this reference for the purpose of illustrating the state of knowledge in the art concerning techniques for drug delivery.
d. "Host" refers to the recipient of the therapy to be practiced according to the invention. The host may be any vertebrate, but will preferably be a mammal. If a mammal, the host will preferably be a human, but may also be a domestic livestock or pet animal.
e. "Target tissue" refers to the tissue of the host in which expression of the polynucleotide is sought.
f. "Skin" as used herein refers to the epidermal, dermal and subcutaneous tissues of a host.
g. "Mucosa" refers to mucosal tissues of a host wherever they may be located in the body including, but not limited to, respiratory passages (including bronchial passages, lung epithelia and nasal epithelia), genital passages (including vaginal, penile and anal mucosa), urinary passages (e.g., urethra, bladder), the mouth, eyes and vocal cords.
h. "Point of Entry" refers to the site of introduction of the polynucleotide into a host, including immediately adjacent tissue.
i. "Dermal" and "Epidermal Administration" mean routes of administration which apply the polynucleotide(s) to or through skin. Dermal routes include intradermal and subcutaneous injections as well as transdermal transmission. Epidermal routes include any means of irritating the outermost layers of skin sufficiently to provoke an immune response to the irritant. The irritant may be a mechanical or chemical (preferably topical) agent.
j. "Epithelial Administration" involves essentially the same method as chemical epidermal administration, except that the chemical irritant is applied to mucosal epithelium.
k. "IL" refers to interleukin and "IFN" refers to interferon.
l. "TH1 Response(s)" refers to a humoral cellular immune response that is induced preferentially by antigens that bind to and activate certain APC's; i.e., macrophages and dendritic cells.

### B. Methods for introduction of the naked gene expression vectors of the invention into target tissues having substantial concentrations of antigen presenting cells: effect of use of naked gene expression vectors on the host immune response.

The method of the invention will be described with respect to the preferred embodiment for use of the naked gene expression vectors of the invention. It will be understood, however, that other recombinant expression vectors may be administered through similar routes, although use of viral expression vectors is not desirable and use of non-naked expression vectors (i.e., with a delivery vehicle) can be expected to significantly reduce the immunostimulatory activity of the immunostimulatory polynucleotides of the invention.

Although the method of the invention is not to be limited by any particular theory regarding the mechanism by which the host immune response is stimulated to provide the host with protection against antigen, the preferred method of the invention (for introduction of antigen-encoding naked gene expression vectors into APCs) is designed to selectively and efficiently boost production of TH1 (helper T cell) lymphocytes for release of IL-12 and to augment CTL activity. In this embodiment, the TH1 component of the T lymphocyte immune response is generally stimulated in preference to the antigenic stimulation of TH2 lymphocytes, which mediate production of IgE antibody.

More specifically, over the last few years it has been shown that CD4+ cells generally fall into one of two distinct subsets, the TH1 and TH2 cells. TH1 cells principally secrete IL-2, IFNγ, IFN-α, IL-12 and TNFβ (the latter two of which mediate macrophage activation and delayed type hypersensitivity) while TH2 cells principally secrete IL-4 (which stimulates production of IgE antibodies), IL-5, IL-6 and IL-10. These CD4+ subsets exert a negative influence on one another; i.e., secretion of TH1 lymphokines inhibits secretion of TH2 lymphokines and vice versa. In addition, it is believed that exposure of TH2 cells to CTLs also suppresses TH2 cell activity.

How the helper T cell subsets are differentially regulated is not completely clear. Factors believed to favor TH1 activation resemble those induced by viral infection and include intracellular pathogens, exposure to IFNγ, IFN-α, and IL-2, the presence of APCs and exposure to low doses of antigen. Factors believed to favor TH2 activation include exposure to IL-4 and IL-10, APC activity on the part of B lymphocytes and high doses of antigen. Active TH1 cells enhance cellular immunity and are therefore of particular value in responding to intracellular infections, while active TH2 cells enhance antibody production and are therefore of value in responding to extracellular infections. However, TH2 cell activity also induces IgE production through the release of IL-4, thus encouraging the formation of IgE-antigen complexes.

In mice, IgG 2A antibodies are serological markers for a TH1 type immune response, whereas IgG 1 antibodies are indicative of a TH2 type immune response. TH2 responses include the allergy-associated IgE antibody class; soluble protein antigens tend to stimulate relatively strong TH2 responses. In contrast, TH1 responses are induced by antigen binding to macrophages and dendritic cells. As shown in the data presented in Examples VI and VII, mice injected intradermally with antigen-encoding polynucleotides preferentially produced IgG 2A antibodies indicative of TH1 responses, which in turn are indicative of the antigen being expressed intracellularly in, then presented by, APCs. In contrast, mice injected intradermally with antigen preferentially produced IgG 1 antibodies indicative of a predominant TH2 cell response.

Thus, administration of naked gene expression vectors which encode antigens (or known immunostimulatory fragments of antigens) according to the invention not only suppresses IgE antibody production, but also does so from the outset of therapy, thus avoiding the risk of anaphylaxis posed by conventional immunotherapy protocols. Specifically, administration of antigen-encoding naked gene expression vectors (particularly through dermal and epidermal routes) selectively stimulates the production of CD4+ TH1 and CD8+ lymphocytes over CD4+ TH2 lymphocytes, stimulates IL-12 and INF-α production, and stimulates INFγ secretion (which suppresses IgE antibody activity).

As reflected in the data presented in Example VI, intradermal challenge with a protein antigen (β galactosidase) selectively induces TH2 responses in mice which, consistent with conventional immunotherapy responses, is gradually replaced by a TH1 response in antigen desensitized mice. However, as demonstrated in Example VII, IgE antibody levels produced in the protein injected mice are substantially greater during the initial phase of treatment than are produced at any stage of treatment of mice injected with a naked gene expression vector (pCMV-LacZ) that operatively encodes the same antigen and includes an immunostimulatory polynucleotide of the invention (SEQ.ID.No.1) Further, in mice challenged with an intradermal dose of the plasmid, the TH1 cell responses greatly exceeded those of TH2 cells. Even more surprisingly, IgE and IL-4 levels in the pCMV-LacZ challenged mice are very low, while antigen-stimulated CTL levels and TH1 cell secretion of interferons are enhanced as compared to protein challenged and control mice. Moreover, the protection against IgE production afforded to the pCMV-LacZ challenged mice continues despite subsequent challenge with the plasmid or protein, even when combined with adjuvant (Examples IV, V and VII).

### C. Methods for introduction of the naked gene expression vectors of the invention into target tissues having substantial concentrations of antigen presenting cells: routes of administration and dosing protocols.

The naked gene expression vectors of the invention may be used as adjuvants in conventional vaccination protocols or may be used in gene immunization protocols; i.e., where the target antigen is a protein antigen encoded by a naked gene expression vector (which may also be the vector that contains the non-coding, immunostimulatory polynucleotides of the invention). The latter approach is preferred and will be discussed in detail below with respect to dosing and administration protocols. Isolated, non-recombinant antigen will be administered according to conventional vaccination techniques.

Many infectious antigens enter the body through the skin or mucosa, where local immunity to such antigens would be of use. For this reason, as well as the relatively high concentration of APCs present in the mammalian skin and mucosa, these tissues are the preferred target tissues of the invention.

For dermal routes of administration, the means of introduction may be by epidermal administration, subcutaneous or intradermal injection. Of these means, epidermal administration is preferred for the greater concentrations of APCs expected to be in intradermal tissue.

The means of introduction for dermal routes of administration which are most preferred, however, are those which are least invasive. Preferred among these means are transdermal transmission and epidermal administration.

For transdermal transmission, iontophoresis is a suitable method. Iontophoretic transmission may be accomplished using commercially available "patches" which deliver their product continuously through unbroken skin for periods of several days or more. Use of this method allows for controlled transmission of pharmaceutical compositions in relatively great concentrations, permits infusion of combination drugs and allows for contemporaneous use of an absorption promoter.

An exemplary patch product for use in this method is the LECTRO PATCH trademarked product of General Medical Company of Los Angeles, CA. This product electronically maintains reservoir electrodes at neutral pH and can be adapted to provide dosages of differing concentrations, to dose continuously and/or to dose periodically. Preparation and use of the patch should be performed according to the manufacturer's printed instructions which accompany the LECTRO PATCH product; those instructions are incorporated herein by this reference.

Epidermal administration essentially involves mechanically or chemically irritating the outermost layer of the epidermis sufficiently to provoke an immune response to the irritant. Specifically, the irritation should be sufficient to attract APC's to the site of irritation. As discussed previously, it is believed that the APC's then take up and express the administered naked polynucleotide.

An exemplary mechanical irritant means employs a multiplicity of very narrow diameter, short tynes which can be used to irritate the skin and attract APC's to the site of irritation, to take up naked polynucleotides transferred from the end of the tynes. For example, the MONO-VACC old tuberculin test manufactured by Pastuer Merieux of Lyon, France contains a device suitable for introduction of naked gene expression vectors of the invention. Another suitable device for use in the invention is a tyne device manufactured for use in allergy testing by Lincoln Diagnostics of Decatur, IL. (and sold under the trademark MULTITEST^{®}).

Such devices typically consist of a plastic container having a syringe plunger at one end and a tyne disk at the other. The tyne disk supports a multiplicity of narrow diameter tynes of a length which will just scratch the outermost layer of epidermal cells. In the present invention, each needle is coated with a pharmaceutical composition of naked gene expression vectors by immersing the tips of the tynes into an aqueous solution of the polynucleotides. For convenience, the tyne device may then be frozen so that the polynucleotide become dried onto the tines and can be administered without having to prepare the device for use at the time of treatment.

Use of the device is according to the manufacturer's written instructions included with the device product; these instructions regarding use and administration are incorporated herein by this reference to illustrate conventional use of the device (see also, Example VII).

Another suitable approach to epidermal administration of naked polynucleotides is by use of a chemical which irritates the outermost cells of the epidermis, thus provoking a sufficient immune response to attract APC's to the area. An example is a keratinolytic agent, such as the salicylic acid used in the commercially available topical depilatory creme sold by Noxema Corporation under the trademark NAIR. This approach may also be used to achieve epithelial administration in the mucosa. The chemical irritant may also be applied in conjunction with the mechanical irritant (as, for example, would occur if the MONO-VACC type tyne were also coated with the chemical irritant). The naked gene expression vector may be suspended in a carrier which also contains the chemical irritant or coadministered therewith (see, Example VIII).

For mucosal administration, the means of introduction will vary according to the location of the point of entry. Particularly for immunization to and treatment of respiratory infections, intranasal administration means are most preferred. These means include inhalation of aerosol suspensions or insufflation of the naked gene expression vectors of the invention. Suppositories and topical preparations will also be suitable for introduction to certain mucosa, such as genital and ocular sites. Also of particular interest with respect to vaginal delivery of naked gene expression vectors of the invention are vaginal sandwich-type rings and pessaries. Examples of these devices and their use are described in Chien, *supra* at Ch.9.

The dosage of each naked gene expression vector to be supplied according to the method of the invention will vary depending on the desired response by the host and the polynucleotide used. Generally, it is expected that up to 100-200 µg of polynucleotide can be administered in a single dosage, although as little as about 0.3 µg of polynucleotide administered through skin or mucosa can induce long lasting immune responses.

For purposes of the invention, however, it is sufficient that the naked gene expression vectors be supplied at a dosage sufficient to cause expression of the antigenic polypeptide encoded by the polynucleotide. These dosages may be modified to achieve therapeutic, subtherapeutic or immunostimulatory levels of expression. Means to confirm the presence and quantity of expressed peptides are well-known to those skilled in the art and will not, therefore, be described in detail. Certain such means are illustrated in the Examples provided below; generally, they include immunoassays (such as enzyme-linked immunosorbent assays), PCR techniques, and immunohistological analyses performed according to techniques which are well known in the art. Dosages of the administered polynucleotides can be adjusted to achieve the desired level of expression based on information provided by these detection and quantification means as well as *in vivo* clinical signs known to practitioners skilled in the clinical arts.

Preferably, naked gene expression vectors of the invention will be administered in in "low" doses (e.g., in mice, about 50µg immunostimulatory polynucleotide or less). Those of ordinary skill in the art will readily be able to determine an equivalent dosage level for use in humans. Those of ordinary skill in the art will be familiar with the course of dosing employed in vaccination and immunotherapy protocols (i.e., priming, booster and maintenance dosing), which course will be suitable for use in the method of the invention. Generally, it can be expected that doses of less than about 50µg immunostimulatory polynucleotide, and even less than about 10µg, will be suitable for priming, booster and maintenance doses in humans. Alternatively, the priming dose of antigen-encoding polynucleotide may be followed by booster and/or maintenance doses of antigen.

Examples illustrating aspects of each embodiment of the invention are provided below. They should be regarded as illustrating rather than limiting the invention, which is defined by the appended claims. Conventional abbreviations (e.g., "ml" for milliliters) are used throughout the Examples.

### EXAMPLE I

### EXPRESSION OF A VIRAL PROTEIN FOLLOWING INTRADERMAL INJECTION OF A NAKED GENE EXPRESSION VECTOR

To demonstrate the competence of naked gene expression vectors of the invention for expression in the dermis, the gene for influenza ribonucleoprotein (NP) was subcloned into a pCMV plasmid. NP genes from numerous strains of influenza are known in the art and are highly conserved in sequence among various strains (see, e.g. Gorman, et al., J. Virol, 65:3704, 1991).

Four eight week old Balb/c mice were injected three times with 15µg of pCMV-RNP suspended in 100 µl of HBSS. Injections were made intradermally at the base of the tails at two week intervals. CTLs recognize antigens presented by class I MHC molecules and play an important role in the elimination of virally infected cells. Intramuscular (i.m.) immunization by means of cDNA expression vectors should be an effective method to introduce antigen into class I MHC molecules and thus stimulate CTL responses. In this study, intradermal (i.d.) injection of a plasmid containing the influenza nucleoprotein (NP) antigen gene induced both NP-specific CTL and high titers of anti-NP antibodies. These antibodies reached a maximum 6 weeks after injection and persisted unchanged for at least 28 weeks, in the absence of local inflammation.

Plasmid DNA was purified by CsCl banding in the presence of ethidium bromide and was stored frozen in 10 mM Tris-HCL, 0.1 mM EDTA, pH 8.0. Before injection, the plasmid was precipitated in ethanol and dissolved in normal saline containing 0.1 mM EDTA.

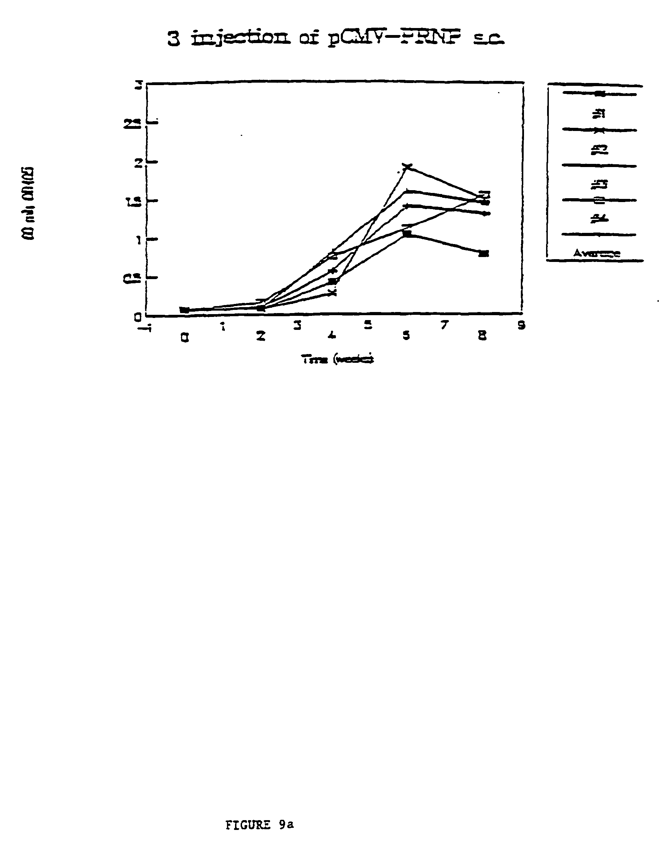

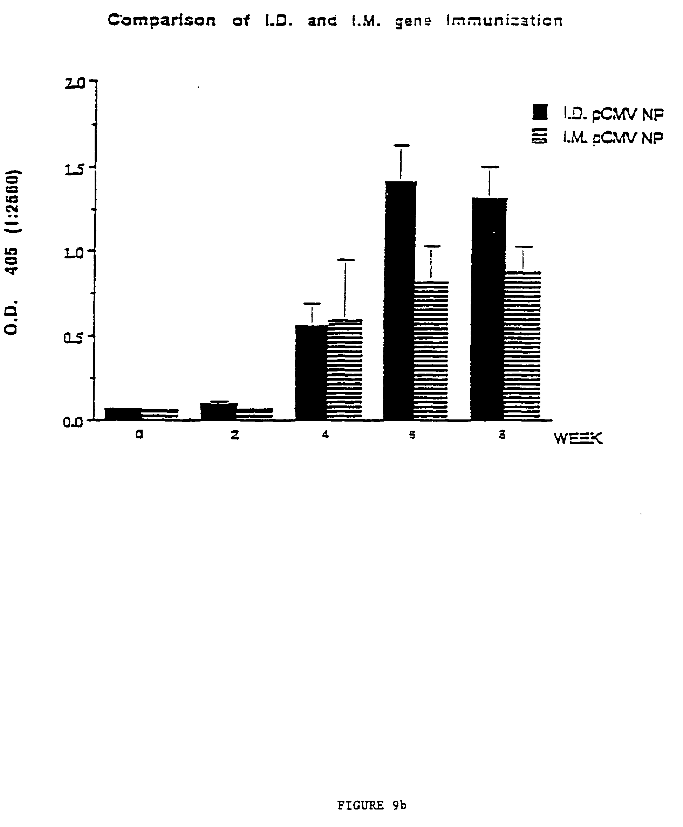

The presence of anti-NP IgG in serum was measured by ELISA substantially as described in Viera, et al., Int. Immunl., 2:487, (1990). The results of this assay are shown in FIGURE 9a; all of the animals developed high titer anti-NP antibodies, which persisted for more than 20 weeks. As shown in FIGURE 9b, the intradermal injections appeared to give about four fold higher antibody titers than intramuscular injections of equivalent amounts of plasmid DNA.

The axes of FIGURES 9a and 9b represent, respectively, the ELISA titer (mean, 1 ounce) against time. Serum dilution for all graph points is 2560.

### EXAMPLE II

### IN VIVO ANTIBODY RESPONSES TO THE IMMUNOSTIMULATORY POLYNUCLEOTIDES OF THE INVENTION

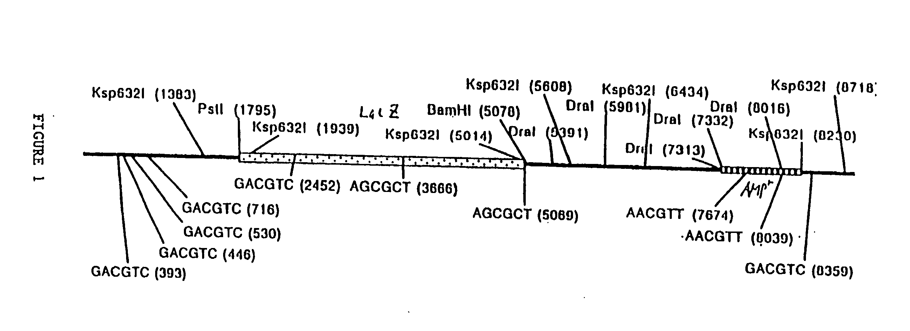

To compare humoral immune responses to naked gene expression vectors containing the immunostimulatory polynucleotides of the invention to humoral immune responses to vectors lacking such polynucleotides, the pCMV-LacZ plasmid described in Example I (which includes two copies of the immunostimulatory polynucleotide of SEQ.ID.No.1) was modified to substitute a gene encoding an enzyme which confers kanamycin resistance (KanR). The resulting plasmid (pKCB-LacZ) lacks any of the immunostimulatory polynucleotides of the invention (see, vector maps in FIGURES 1 [pCMV-LacZ] and 3 [pKCB-LacZ]). In contrast, the AmpR containing pCMV-LacZ plasmid includes the AACGTT (SEQ.ID.No.1) palindromic sequence at two separate locations in the vector within the AmpR gene.

Four Balb/c mice per group were each injected intradermally at the base of the tail with 50µg of either the pCMV-LacZ or pKCB-LacZ plasmids. Each injection was repeated twice at one week intervals. A third group of mice was injected with pKCB-LacZ and supplementally injected with pUC-19, a plasmid which includes the AmpR gene. As a control, a fourth group of mice was injected with a non-specific bacterial DNA. For comparison of the overall immune response elicited, a fifth group was injected with a naked gene expression vector which operatively encodes GM-CSF (granulocyte-monocyte colony stimulating factor). Anti-antigen antibody production was measured by serum ELISA after 6 weeks.

As shown in FIGURE 2, the mice injected with pCMV-LacZ produced antibodies against the expressed LacZ reporter molecule. However, no antibody formation was detected in the sera of the mice who received the pKCB-LacZ plasmid, despite the higher level of LacZ expression achieved by the vector (detected as a measure of β-galactosidase activity in Chinese hamster ovary cells transfected separately with each vector; see, FIGURE 10). Yet anti-LacZ antibody production was restored with co-administration of pKCB-LacZ and pUC-19 (FIGURE 5), although no such response was detected after injection of the control plasmid (id.). The enhancing effect of the pUC-19 vector exceeded even the response to the GM-CSF encoding vector (id.).

To determine the effect of the immunostimulatory polynucleotides of the invention on humoral immune responses, the pKCB-LacZ plasmid was modified to include one or two copies of the AACGTT polynucleotide palindrome found in the AmpR gene (pKCB-1aaZ [1 copy] and pKCB-2aaZ [2 copies]). For comparison, groups of pKCB-LacZ and pCMV-LacZ injected mice were also injected with, respectively, KCB or CMV plasmids which lacked the LacZ reporter molecule. Antibody responses to LacZ were measured at 4 weeks after 3 weeks of immunization as described above.

As shown in FIGURE 4, virtually no antibody response to LacZ was measured in the mice injected with pKCB-LacZ or pKCB-LacZ/pKCB, while antibody responses were detected in the mice injected with pCMV-LacZ and pCMV-LacZ/pCMV. Moreover, the mice injected with the modified KCB plasmids produced substantially greater antibody titers than even the mice injected with the pCMV plasmids, which responses increased in proportion to the number of copies of the AACGTT polynucleotide (SEQ.ID.No.1) present in the plasmid. The enhanced response as compared to the pCMV plasmids (which contain two copies of the AACGTT polynucleotide) is probably attributable to the greater levels of antigen expression achieved by the KCB vectors (see, FIGURE 10).

### EXAMPLE III

### IN VIVO CTL ACTIVITY IN RESPONSE TO TO THE IMMUNOSTIMULATORY POLYNUCLEOTIDES OF THE INVENTION

To determine whether the immunostimulatory polynucleotides of the invention (i.e., palindromic, CG containing sequences) stimulate cellular as well as humoral responses, the lytic activity of CTLs after immunization of mice with either pKCB-LacZ or pCMV-LacZ was tested. A control group of mice was immunized with the antigen in alum.

36 weeks after immunization (performed as described in Example II), the mice were sacrificed and splenocytes were removed for use in standard mixed lymphocyte cultures. The cultures were grown in the presence of a known synthetic β-galactosidase peptide. The cultures were assayed for anti-LacZ CTL activity 5-6 days, measured as a function of the percent lysis of cells exposed to the antigen by pulsing versus the effector (antigen):target ratio.

As shown in FIGURE 6, as the effector:target ratio was increased, the CTL activity in cultures of cells from the pCMV-LacZ injected mice increased from about 18% to nearly 100%. In contrast, the CTL activity in cultures from the pKCB-LacZ and control injected mice barely exceeded 20% lytic activity even when the effector:target ratio was raised to 36:1.

To determine the effect of the two copies of the immunostimulatory polynucleotide (AACGTT) of SEQ.ID.No.1 in the pCMV-LacZ plasmid, another group of pKCB-LacZ injected mice received a co-injection of either 5µg or 100µg of pUC-19. An increase in CTL activity to nearly 60% lysis was achieved in the latter group (FIGURE 7).

### EXAMPLE IV

### IMMUNE RESPONSE TO VIRAL CHALLENGE BY MICE INTRADERMALLY INJECTED WITH NAKED GENE EXPRESSION VECTORS CONTAINING IMMUNOSTIMULATORY POLYNUCLEOTIDES OF THE INVENTION

To test whether immunity generated by vaccination with naked gene expression vectors of the invention could protect animals from a lethal viral challenge, groups of 10 Balb/c mice were injected intradermally 3 times with 15 µg of a pCMV plasmid (pCMV-NP) which contained two copies of the immunostimulatory polynucleotide of SEQ.ID.No. 1 and the NP gene from an H1N1 strain of influenza virus (A/PR/8/34; provided by Dr. Inocent N. Mbawvike at the Baylor College of Medicine, U.S.) Control groups included uninjected animals as well as animals injected with an irrelevant plasmid (pnBL3) .

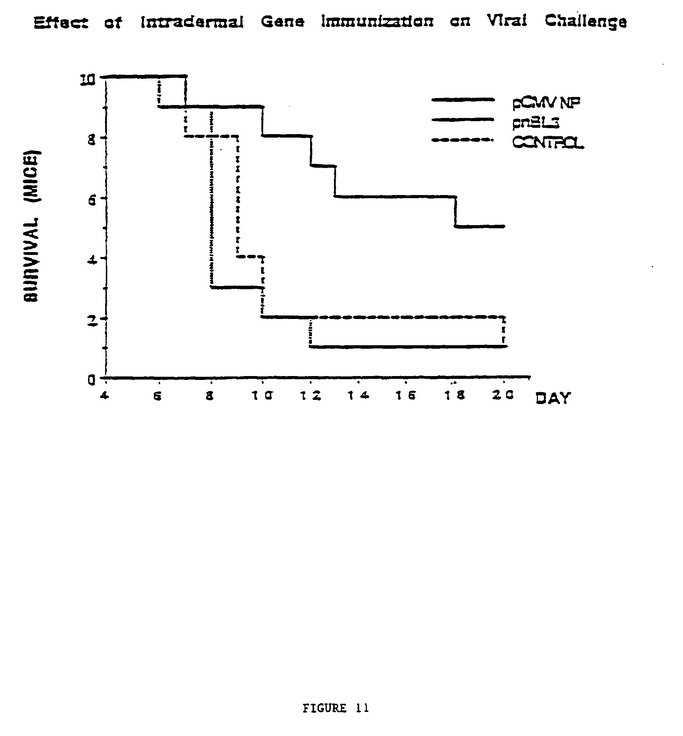

Six weeks after the initial plasmid injections, the animals were challenged with a LD₉₀ dose of an H3N2 influenza strain (A/HK/68); also provided by Dr. Mbawuike). Intradermally vaccinated mice were significantly protected from the challenge (P(0.01) as compared to unvaccinated control mice; see, FIGURE 11 (a Kaplan-Meyer survival curve).

### EXAMPLE V

### PROLONGED IMMUNOLOGIC MEMORY AFTER INTRADERMAL ADMINISTRATION OF NAKED POLYNUCLEOTIDES INDUCED BY ANTIGEN STIMULATION OF T CELLS

To test whether the protective effect observed in the mice described in Example IV included long-term immunologic protective memory, 0.1, 1, 10 and 100 µg of naked gene expression vectors (0.5-5 ng/l mg DNA endotoxin content) encoding the E.coli enzyme β-galactosidase under the control of the CMV promoter were administered to groups of 4 mice\dosage\route either intramuscularly ("IM") or intradermally ("ID"). Each plasmid included two copies of the immunostimulatory polynucleotide of SEQ.ID.No.1 (pCMV-LacZ).

As a control, another group of 4 mice\dosage received 100 µg β-galactosidase protein ("PR") intradermally. All injections were made using 50 µl normal saline as carrier. IM and ID injections were made with a 0.5 ml syringe and a 28.5 gauge needle. Antibodies were thereafter measured by enzyme-linked immunoabsorbent assay at 2 week intervals.

Total anti-β galactosidase antibodies were measured using β-galactosidase (Calbiochem, CA) as the solid phase antigen. Microtiter plates (Costar, Cambridge, MA) were coated with 5 µg of antigen dissolved in 90mM borate (pH 8.3) and 89mM NaCl (i.e., borate buffered saline; BBS) overnight at room temperature and blocked overnight with 10 mg/ml of bovine serum albumin in BBS.

Serum samples were serially diluted in BBS starting at a 1:40 dilution for the first 8 weeks, them a 1:320 dilution thereafter. These samples were added to the plates and stored overnight at room temperature. Plates were washed in BBS+0.05% polysorbate 20, then reacted with a 1:2000 dilution of alkaline phosphatase labeled goat anti-mouse IgG antibody (Jackson Immunoresearch Labs., West Grove, PA) for 1 hour at room temperature, or were reacted with a 1:2000 dilution of alkaline phosphatase labeled goat anti-mouse IgG 1 antibody (Southern Biotech of AL), or were reacted with a 1:500 dilution of alkaline phosphatase labled rat anti-mouse IgG 2A antibody (Pharmingen, of CA), under the same conditions. Plates were washed again, then a solution of 1 mg/ml of p-nitrophenol phosphate (Boehringer-Mannheim, Indianapolis, IN) in 0.05 M carbonate buffer (pH 9.8), containing 1mM MgCl₂ was added. Absorbance at 405 nm was read 1 hour after addition of substrate to the plates.

Lesser antibody responses were measured in the animals who had received the pCMV Lac-Z plasmids by IM injection than by ID injection (data not shown).

To assess for T cell memory, the animals were then boosted with 0.5 µg of PR at a separate site by ID injection. If these animals had developed memory T cells to control production of antibody to β-galactosidase, they would be expected to mount a more vigorous immune response after boosting with soluble protein antigen than had been demonstrated in response to the priming dose of antigen.

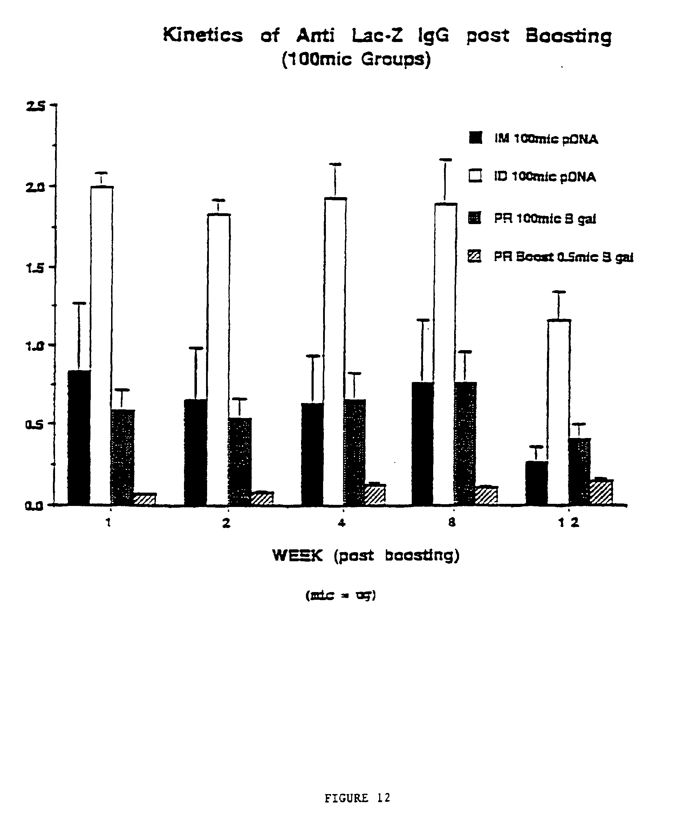

As shown in FIGURE 12, it is clear that the animals which had received ID injections of pCMV-LacZ plasmid had developed substantially better immunological memory than did animals which had received either IM injections of plasmid or of PR. Further, the memory which was developed by the ID injected animals persisted for a minimum of about 12 weeks.

### EXAMPLE VI

### SELECTIVE INDUCTION OF A TH1 RESPONSE AFTER INTRADERMAL ADMINISTRATION OF NAKED POLYNUCLEOTIDES

In mice, IgG 2A antibodies are serological markers for a TH1 type immune response, whereas IgG 1 antibodies are indicative of a TH2 type immune response. TH2 responses include the allergy-associated IgE antibody class; soluble protein antigens tend to stimulate relatively strong TH2 responses. In contrast, TH1 responses are induced by antigen binding to macrophages and dendritic cells. TH1 responses are to be of particular importance in the treatment of allergies and AIDS.

To determine which response, if any, would be produced by mice who received naked gene expression vectors according to the invention, mice were vaccinated with the pCMV-LacZ vector described in Example V or protein as described in Example V. At 2 week intervals, any IgG 2a and IgG 1 to β-galactosidase were measured by enzyme-linked immunoabsorbent assay (using antibodies specific for the IgG 1 and IgG 2A subclasses) on microtiter plates coated with the enzyme.

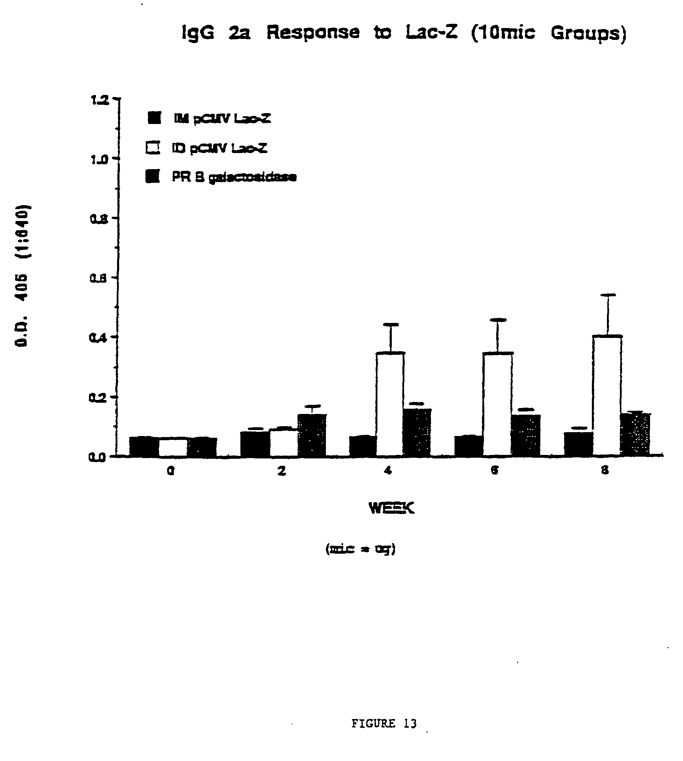

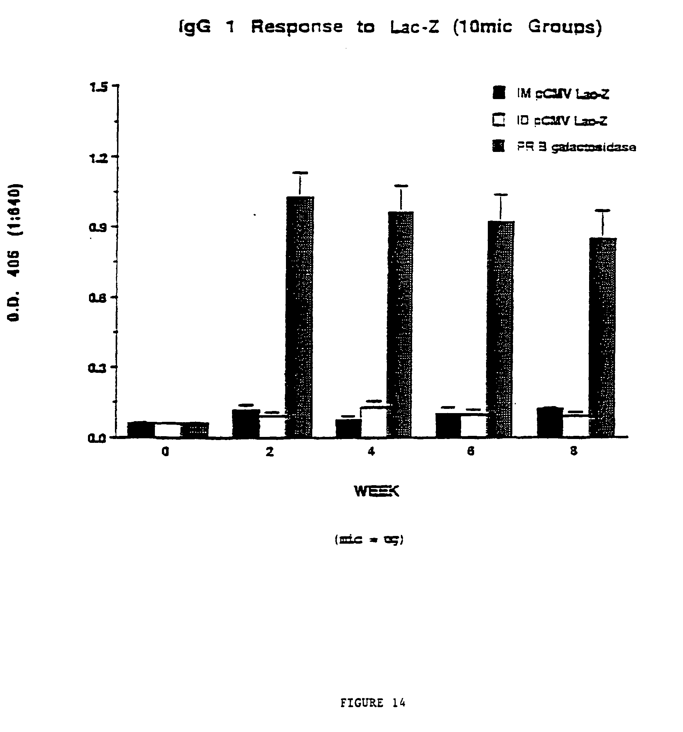

As shown in FIGURE 13, only the mice who received the plasmid by ID injection produced high titers of IgG 2A antibodies. As shown in FIGURE 14, immunization of the mice with the enzyme itself ("PR") induced production of relatively high titers of IgG 1 antibodies. In the IM injected mice, low titers of both IgG 2A and IgG 1 antibodies were produced without apparent selectivity. The data shown in the FIGURES comprise averages of the values obtained from each group of 4 mice.

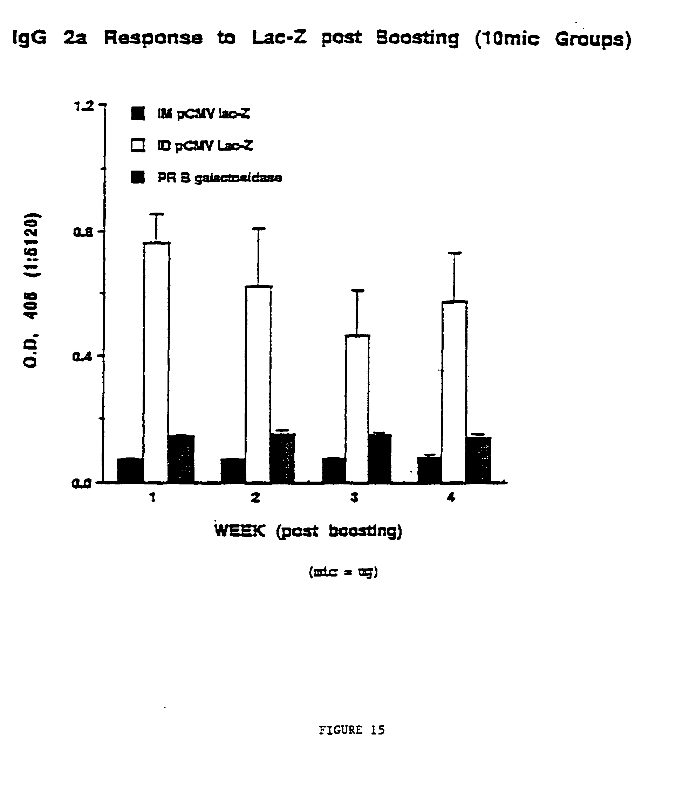

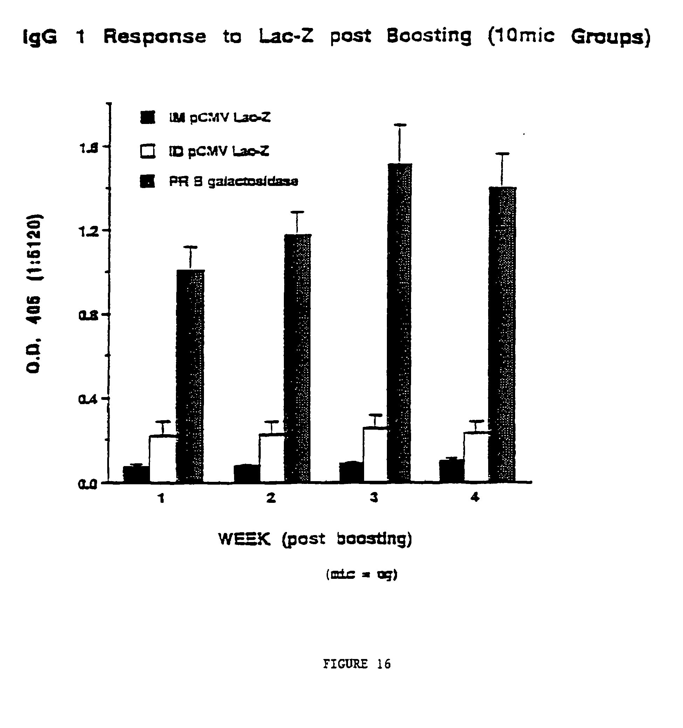

To determine the stability of the antibody response over time, the same group of animals were boosted with 0.5 µg of enzyme injected intradermally. As shown in FIGURES 15 and 16 boosting of ID injection primed animals with the enzyme induced a nearly 10-fold rise in IgG 2A antibody responses (i.e., the antibody titer rose from 1:640 to 1:5120), but did not stimulate an IgG 1 response. These data indicate that the selective TH1 response induced by ID administration of naked polynucleotides is maintained in the host, despite subsequent exposure to antigen.

### EXAMPLE VII

### SUPPRESSION OF IgE ANTIBODY RESPONSE TO ANTIGEN BY IMMUNIZATION WITH ANTIGEN-ENCODING POLYNUCLEOTIDES

Using the experimental protocol described in Examples V and VI, five to eight week old Balb/c mice were immunized with one of two naked gene expression vectors of the invention: the pCMV-LacZ plasmid described in Example V or a control plasmid, pCMV-BL (which does not encode for any insert peptide and does not contain immunostimulatory polynucleotides). A third group of the mice received injections of antigen (β galactosidase). Plasmid DNA was purified and its endotoxin content reduced to 0.5-5ng/lmg DNA by extraction with TRITON X-114 (Sigma, St. Louis, MI). Before inoculation, pDNA was precipitated in ethanol, washed with 70% ethanol and dissolved in pyrogen free normal saline.

Immunization was by intradermal injection of plasmid DNA loaded onto separate tynes of a MONOVACC^{®} multiple tyne device (Connaught Lab, Inc., Swiftwater, PA). Briefly, the tyne devices were prepared after extensive washing in DDW and overnight soaking in 0.5% SDS (sulfated dodecyl saline), washed again in DDW, soaked overnight in 0.1N NaOH, washed again in DDW and dried at 37°C for 8 hours. Six µl of plasmid DNA dissolved in normal saline were pipetted onto the tynes of the tyne device just prior to each inoculation described below. The total amount of pDNA loaded on the device per inoculation was 25 µg each of pCMV-LacZ and pCMV-BL. For purposes of estimating actual doses, it was assumed that less than 10% of the pDNA solution loaded onto the tyne device was actually introduced on injection of the tynes into intradermal tissue.

Each mouse was treated 3 times with 2 inoculations of each plasmid in a one week interval injected intradermally at the base of the tail. Another group of mice received a single intradermal injection in the base of the tail of 10µg of β galactosidase protein (dissolved in 50µl of normal saline) in lieu of pDNA.

Toward inducing an IgE antibody response to subsequent antigen challenge, each group of mice was injected once intraperitoneally with 0.1 ml of phosphate buffered saline (PBS) solution containing 1µg of antigen (β galactosidase; Calbiochem, San Diego, CA) and 3mg of ALUM aluminum hydroxide as adjuvant (Pierce Chemical, Rockford, IL) 14 weeks after the initial immunization. Total IgE was assayed in sera from the mice 4 times over the subsequent 4 consecutive weeks.

IgE was detected using a solid phase radioimmunoassay (RAST) in a 96 well polyvinyl plate (a radioisotopic modification of the ELISA procedure described in Coligan, "Current Protocols In Immunology", Unit 7.12.4, Vol. 1, Wiley & Sons, 1994), except that purified polyclonal goat antibodies specific for mouse e chains were used in lieu of antibodies specific for human Fab. To detect anti-LacZ IgE, the plates were coated with β galactosidase (10µg/ml). The lowest IgE concentration measurable by the assay employed was 0.4ng of IgE/ml.

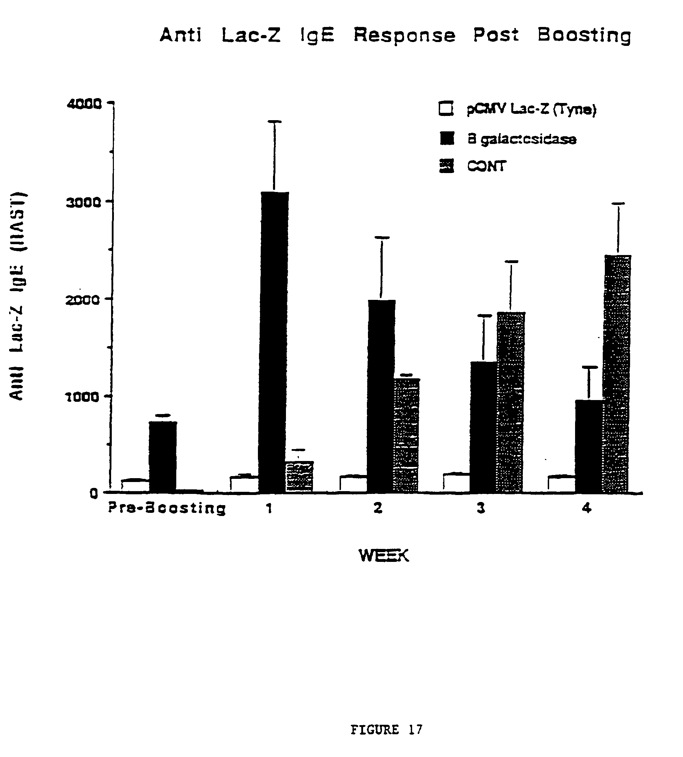

Measuring specifically the anti-antigen response by each group of mice, as shown in FIGURE 17, anti-LacZ IgE levels in the plasmid injected mice were consistently low both before and after boosting (averaging about 250 CPM in RAST), while the protein injected mice developed high levels of anti-LacZ, particularly after the first antigen booster injection, when anti-LacZ levels in the mice rose to an average of about 3000 CPM. Consistent with acquisition of tolerance, anti-LacZ IgE levels in the protein injected mice declined over time, but continued to rise in the control mice who had not received any immunization to β galactosidase.

These data show that the plasmid injected mice developed an antigen specific TH1 response to the plasmid expression product, with concomitant suppression of IgE production, while tolerance was acquired in the protein injected mice only after development of substantially higher levels of total and antigen specific IgE antibodies.

### EXAMPLE VIII

### EPIDERMAL ADMINISTRATION OF A NAKED GENE EXPRESSION VECTOR USING A CHEMICAL AGENT TO ELICIT AN IMMUNE RESPONSE

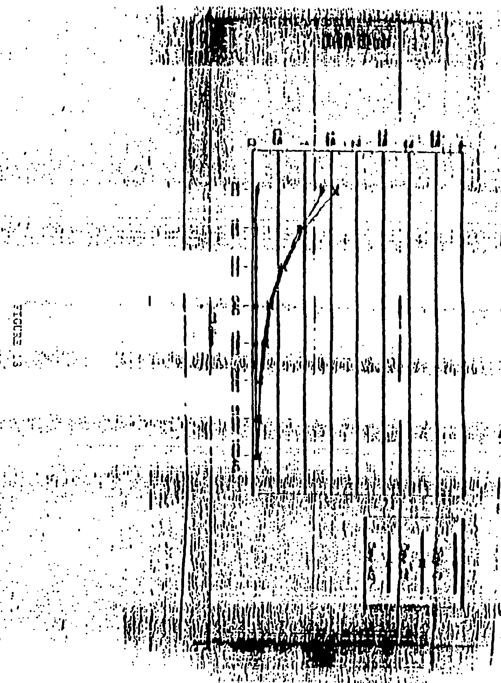

FIGURE 18 depicts the results of an ELISA performed as described in Example I for serum levels of anti-NP IgG following epidermal administration of the pCMV-NP vector described in Example I in conjunction with the application of a chemical agent.

The plasmid was suspended in 40 µg of an isotonic normal saline solution containing approximately 150 µg of plasmid per milliliter. This solution was absorbed onto the nonadhesive pad of a BAND-AID^{®} brand bandage (Johnson & Johnson).

A Balb/c mouse was shaved along the base of its tail and a commercially available keratinolytic agent (here, the previously described depilatory creme sold under the trademark NAIR^{®}) was applied to the shaved skin. After several minutes, the keratinolytic agent was washed off of the skin and the plasmid-containing bandage applied thereto. As shown in FIGURE 18, the treated animal developed serum anti-NP IgG at a titer of 1:640.

### EXAMPLE IX

### ENHANCEMENT OF INTERFERON AND CYTOKINE (IL-4) PRODUCTION IN ANIMALS IMMUNIZD WITH IMMUNOSTIMULATORY POLYNUCLEOTIDE CONTAINING PLASMIDS

Two groups of mice were immunized with either pCMV-LacZ or pKCB-LacZ as described in Example III. A third group of mice received a combination dose of pKCB-LacZ and pUC-19 as described in Example II. After sacrifice, splenocytes were removed and challenged in vitro with β-galactosidase antigen. The release of IFN-γ and IL-4 into supernatants from the antigen challenged cells was measured.

Mice immunized with pKCB-LacZ alone produced little IFN-γ and IL-4 as compared to mice immunized with pCMV-LacZ or the combination pKCB-LacZ/pUC-19 dose (see, FIGURES 19 and 20).

### SUMMARY OF SEQUENCES

SEQ.ID.No.1 is a non-coding, immunostimulatory polynucleotide useful in the invention.
SEQ.ID.No.2 is a non-coding, immunostimulatory polynucleotide useful in the invention.
SEQ.ID.No.3 is a non-coding, immunostimulatory polynucleotide useful in the invention.
SEQ.ID.No.4 is a non-coding, immunostimulatory polynucleotide useful in the invention.
SEQ.ID.No.5 is a non-coding, immunostimulatory polynucleotide useful in the invention.
SEQ.ID.No.6 is a non-coding, immunostimulatory polynucleotide useful in the invention.
SEQ.ID.No.7 is a non-coding, immunostimulatory polynucleotide useful in the invention.
SEQ.ID.No.8 is a non-coding, immunostimulatory polynucleotide useful in the invention.
SEQ.ID.No.9 is a non-coding, immunostimulatory polynucleotide useful in the invention.
SEQ.ID.No.10 is a non-coding, immunostimulatory polynucleotide useful in the invention.
SEQ.ID.No.11 is a non-coding, immunostimulatory polynucleotide useful in the invention.
SEQ.ID.No.12 is a non-coding, immunostimulatory polynucleotide useful in the invention.
SEQ.ID.No.13 is a non-coding, immunostimulatory polynucleotide useful in the invention.
SEQ.ID.No.14 is a non-coding, immunostimulatory polynucleotide useful in the invention.
SEQ.ID.No.15 is a non-coding, immunostimulatory polynucleotide useful in the invention.
SEQ.ID.No.16 is a non-coding, immunostimulatory polynucleotide useful in the invention.
SEQ.ID.No.17 is a non-coding, immunostimulatory polynucleotide useful in the invention.
SEQ.ID.No.18 is a non-coding, immunostimulatory polynucleotide useful in the invention.
SEQ.ID.No.19 is a non-coding, immunostimulatory polynucleotide useful in the invention.
SEQ.ID.No.20 is a non-coding, immunostimulatory polynucleotide useful in the invention.

## Claims

1. The use of an immunostimulatory polynucleotide comprising the sequence 5'-cytosine-guanine-3', in the preparation of a medicament wherein the medicament further comprises an antigen, for use in the treatment by administration to the mucosa of a condition requiring stimulation of a Th1 lymphocyte response to said antigen in a mammal, wherein the condition is an infectious disease.

2. The use of claim 1 wherein the infectious disease is viral and the antigen is a viral antigen.

3. The use of claim 1 or 2 wherein the polynucleotide further encodes the antigen.

4. The use of claim 1 or 2 wherein the polynucleotide is non-replicating.

5. The use of claim 1 or 2 wherein the polynucleotide is non-coding.

6. The use of claim 1 or 2 wherein the antigen is a polypeptide.

7. The use of any one of claims 1 to 6 wherein the immunostimulatory polynucleotide is at least 6 nucleotides in length.

8. The use of any one of claims 1 to 7, wherein the immunostimulatory polynucleotide comprises the sequence 5'-purine-purine-C-G-pyrimidine-pyrimidine-3'.

9. The use of any one of claims 1 to 7, wherein the immunostimulatory polynucleotide comprises the sequence 5'-pyrimidine-pyrimidine-C-G-purine-purine-3'.

10. The use of any one of claims 1 to 7, wherein the immunostimulatory polynucleotide comprises the sequence (TCG)ₙ, where n = 1 to 3.

11. The use of any one of claims 1 to 7, wherein the immunostimulatory polynucleotide comprises a sequence selected from the group consisting of GCGCGC, AGCGCT, CGATCG, CGCGCG, ACCGGT, GACGATCGTC, CGACGATCGTCG, CGACGACGATCGTCGTCG, CAACGTTG, AACAACGTTGTT, CAACAACGTTGTTG, GACGTC, ATCGAT, CGTACG, TCGCGA, ACGT, ACGATCGT, ACAACGTTGT and AACGTT.

12. The use of any one of claims 1 to 11 wherein the mammal is a human.

13. The use of any one of claims 1 to 12 wherein the immunostimulatory polynucleotide is in a plasmid.

14. The use of any one of claims 1 to 12 wherein the immunostimulatory polynucleotide is in a cosmid.

15. A composition comprising an antigen and an immunostimulatory polynucleotide comprising the sequence 5'-cytosine-guanine-3', for use in a treatment of a condition requiring stimulation of a Th1 lymphocyte response to said antigen wherein the condition is an infectious disease in a mammal and wherein said composition is administered to the mucosa.

16. A composition for the use of claim 15 wherein the infectious disease is viral and the antigen is a viral antigen.

17. A composition for the use of claim 15 or 16 wherein the polynucleotide further encodes the antigen.

18. A composition for the use of claim 15 or 16 wherein the polynucleotide is non-replicating.

19. A composition for the use of claim 15 or 16 wherein the polynucleotide is non-coding.

20. A composition for the use of claim 15 or 16 wherein the antigen is a polypeptide.

21. A composition for the use of any one of claims 15 to 20 wherein the immunostimulatory polynucleotide is at least 6 nucleotides in length.

22. A composition for the use of any one of claims 15 to 21, wherein the immunostimulatory polynucleotide comprises the sequence 5'-purine-purine-C-G-pyrimidine-pyrimidine-3'.

23. A composition for the use of any one of claims 15 to 21, wherein the immunostimulatory polynucleotide comprises the sequence 5'-pyrimidine-pyrimidine-C-G-purine-purine-3'.

24. A composition for the use of any one of claims 15 to 21, wherein the immunostimulatory polynucleotide comprises the sequence (TCG)ₙ, where n = 1 to 3.

25. A composition for the use of any one of claims 15 to 21, wherein the immunostimulatory polynucleotide comprises a sequence selected from the group consisting of GCGCGC, AGCGCT, CGATCG, CGCGCG, ACCGGT, GACGATCGTC, CGACGATCGTCG, CGACGACGATCGTCGTCG, CAACGTTG, AACAACGTTGTT, CAACAACGTTGTTG, GACGTC, ATCGAT, CGTACG, TCGCGA, ACGT, ACGATCGT, ACAACGTTGT and AACGTT.

26. A composition for the use of any one of claims 15 to 25 wherein the mammal is a human.

27. A composition for the use of any one of claims 15 to 26 wherein the immunostimulatory polynucleotide is in a plasmid.

28. A composition for the use of any one of claims 15 to 26 wherein the immunostimulatory polynucleotide is in a cosmid.

## Patentansprüche

1. Verwendung eines immunstimulatorischen Polynucleotids, umfassend die Sequenz 5'-Cytosin-Guanin-3', zur Herstellung eines Medikaments, worin das Medikament weiters ein Antigen umfasst, zur Verwendung in der Behandlung einer Erkrankung, die eine Stimulierung einer Th1-Lymphozyten-Antwort auf das Antigen bei einem Säugetier erfordert, durch die Verabreichung an die Mucosa, wobei die Erkrankung eine Infektionskrankheit ist.

2. Verwendung nach Anspruch 1, worin die Infektionskrankheit viral ist und das Antigen ein virales Antigen ist.

3. Verwendung nach Anspruch 1 oder 2, worin das Polynucleotid weiters für das Antigen kodiert.

4. Verwendung nach Anspruch 1 oder 2, worin das Polynucleotid nicht-replizierend ist.

5. Verwendung nach Anspruch 1 oder 2, worin das Polynucleotid nicht-kodierend ist.

6. Verwendung nach Anspruch 1 oder 2, worin das Antigen ein Polypeptid ist.

7. Verwendung nach einem der Ansprüche 1 bis 6, worin das immunstimulatorische Polynucleotid zumindest 6 Nucleotide lang ist.

8. Verwendung nach einem der Ansprüche 1 bis 7, worin das immunstimulatorische Polynucleotid die Sequenz 5'-Purin-Purin-C-G-Pyrimidin-Pyrimidin-3' umfasst.

9. Verwendung nach einem der Ansprüche 1 bis 7, worin das immunstimulatorische Polynucleotid die Sequenz 5'-Pyrimidin-Pyrimidin-C-G-Purin-Purin-3' umfasst.

10. Verwendung nach einem der Ansprüche 1 bis 7, worin das immunstimulatorische Polynucleotid die Sequenz (TCG)ₙ umfasst, worin n = 1 bis 3 ist.

11. Verwendung nach einem der Ansprüche 1 bis 7, worin das immunstimulatorische Polynucleotid eine Sequenz umfasst, die aus der aus GCGCGC, AGCGCT, CGATCG, CGCGCG, ACCGGT, GACGATCGTC, CGACGATCGTCG, CGACGACGATCGTCGTCG, CAACGTTG, AACAACGTTGTT, CAACAACGTTGTTG, GACGTC, ATCGAT, CGTACG, TCGCGA, ACGT, ACGATCGT, ACAACGTTGT und AACGTT bestehenden Gruppe ausgewählt ist.

12. Verwendung nach einem der Ansprüche 1 bis 11, worin das Säugetier ein Mensch ist.

13. Verwendung nach einem der Ansprüche 1 bis 12, worin das immunstimulatorische Polynucleotid in einem Plasmid vorliegt.

14. Verwendung nach einem der Ansprüche 1 bis 12, worin das immunstimulatorische Polynucleotid in einem Cosmid vorliegt.

15. Zusammensetzung, umfassend ein Antigen und ein immunstimulatorisches Polynucleotid, umfassend die Sequenz 5'-Cytosin-Guanin-3', zur Verwendung in einer Behandlung einer Krankheit, welche die Stimulierung einer Th1-Lymphozyten-Antwort auf das Antigen erfordert, worin die Krankheit eine Infektionskrankheit bei einem Säugetier ist und worin die Zusammensetzung an die Mucosa verabreicht wird.

16. Zusammensetzung zur Verwendung nach Anspruch 15, worin die Infektionskrankheit viral ist und das Antigen ein virales Antigen ist.

17. Zusammensetzung zur Verwendung nach Anspruch 15 oder 16, worin das Polynucleotid weiters für das Antigen kodiert.

18. Zusammensetzung zur Verwendung nach Anspruch 15 oder 16, worin das Polynucleotid nicht-replizierend ist.

19. Zusammensetzung zur Verwendung nach Anspruch 15 oder 16, worin das Polynucleotid nicht-kodierend ist.

20. Zusammensetzung zur Verwendung nach Anspruch 15 oder 16, worin das Antigen ein Polypeptid ist.

21. Zusammensetzung zur Verwendung nach einem der Ansprüche 15 bis 20, worin das immunstimulatorische Polynucleotid zumindest 6 Nucleotide lang ist.

22. Zusammensetzung zur Verwendung nach einem der Ansprüche 15 bis 21, worin das immunstimulatorische Polynucleotid die Sequenz 5'-Purin-Purin-C-G-Pyrimidin-Pyrimidin-3' umfasst.

23. Zusammensetzung zur Verwendung nach einem der Ansprüche 15 bis 21, worin das immunstimulatorische Polynucleotid die Sequenz 5'-Pyrimidin-Pyrimidin-C-G-Purin-Purin-3' umfasst.

24. Zusammensetzung zur Verwendung nach einem der Ansprüche 15 bis 21, worin das immunstimulatorische Polynucleotid die Sequenz (TCG)ₙ umfasst, worin n = 1 bis 3 ist.

25. Zusammensetzung zur Verwendung nach einem der Ansprüche 15 bis 21, worin das immunstimulatorische Polynucleotid eine Sequenz umfasst, die aus der aus GCGCGC, AGCGCT, CGATCG, CGCGCG, ACCGGT, GACGATCGTC, CGACGATCGTCG, CGACGACGATCGTCGTCG, CAACGTTG, AACAACGTTGTT, CAACAACGTTGTTG, GACGTC, ATCGAT, CGTACG, TCGCGA, ACGT, ACGATCGT, ACAACGTTGT und AACGTT bestehenden Gruppe ausgewählt ist.

26. Zusammensetzung zur Verwendung nach einem der Ansprüche 15 bis 25, worin das Säugetier ein Mensch ist.

27. Zusammensetzung zur Verwendung nach einem der Ansprüche 15 bis 26, worin das immunstimulatorische Polynucleotid in einem Plasmid vorliegt.

28. Zusammensetzung zur Verwendung nach einem der Ansprüche 15 bis 26, worin das immunstimulatorische Polynucleotid in einem Cosmid vorliegt.

## Revendications

1. Utilisation d'un polynucléotide immunostimulatoire comprenant la séquence 5'-cytosine-guanine-3', pour la préparation d'un médicament, où le médicament comprend en outre un antigène, pour utilisation dans le traitement par l'administration à la muqueuse d'une pathologie nécessitant la stimulation d'une réponse de lymphocytes Th1 audit antigène dans un mammifère, où l'état pathologique est une maladie infectieuse.

2. Utilisation selon la revendication 1, où la maladie infectieuse est virale, et l'antigène est un antigène viral.

3. Utilisation de la revendication 1 ou 2, où le polynucléotide code en outre pour l'antigène.

4. Utilisation de la revendication 1 ou 2, où le polynucléotide est non-répliquant.

5. Utilisation de la revendication 1 ou 2, où le polynucléotide est non-codant.

6. Utilisation de la revendication 1 ou 2, où l'antigène est un polypeptide.

7. Utilisation selon l'une quelconque des revendications 1 à 6, où le polynucléotide immunostimulatoire a au moins 6 nucléotides en longueur.

8. Utilisation selon l'une quelconque des revendications 1 à 7, où le polynucléotide immunostimulatoire comprend la séquence 5'-purine-purine-C-G-pyrimidine-pyrimidine-3'.

9. Utilisation selon l'une quelconque des revendications 1 à 7, où le polynucléotide immunostimulatoire comprend la séquence 5'-pyrimidine-pyrmidine-C-G-purine-purine-3'.

10. Utilisation selon l'une quelconque des revendications 1 à 7, où le polynucléotide immunostimulatoire comprend la séquence (TCG)ₙ, où n = 1 à 3.

11. Utilisation selon l'une quelconque des revendications 1 à 7, où le polynucléotide immunostimulatoire comprend une séquence sélectionnée dans le groupe consistant en GCGCGC, AGCGCT, CGATTCG, CGCGCG, ACCGGT, GACGATCGTC, CGACGATCGTCG, CGACGACGATCGTCGTCG, CAACGTTG, CAACAACGTTGTTG, GACGTC, ATCGAT, CGTACG, TCCGCGA, ACGT, ACGATCGT, ACAACGTTGT et AACGTT.

12. Utilisation selon l'une quelconque des revendications 1 à 11, où le mammifère est un être humain.

13. Utilisation selon l'une quelconque des revendications 1 à 12, où le polynucléotide immunostimulatoire est dans un plasmide.

14. Utilisation selon l'une quelconque des revendications 1 à 12, où le polynucléotide immunostimulatoire est dans un cosmide.

15. Composition comprenant un antigène et un polynucléotide immunostimulatoire comprenant la séquence 5'-cytosine-guanine-3', pour utilisation dans un traitement d'un état pathologique nécessitant la stimulation d'une réponse de lymphocyte Th1 audit antigène, où l'état pathologique est une maladie infectieuse dans un mammifère, et où ladite composition est administrée à la muqueuse.

16. Composition pour l'utilisation selon la revendication 15, où la maladie infectieuse est virale, et l'antigène est un antigène viral.

17. Composition pour l'utilisation selon la revendication 15 ou 16, où le polynucléotide code en outre pour l'antigène.

18. Composition pour l'utilisation selon la revendication 15 ou 16, où le polynucléotide est non-repliquant.

19. Composition pour l'utilisation selon la revendication 15 ou 16, où le polynucléotide est non-codant.

20. Composition pour l'utilisation selon la revendication 15 ou 16, où l'antigène est un polypeptide.

21. Composition pour l'utilisation selon l'une quelconque des revendications 15 à 20, où le polynucléotide immunostimulatoire a au moins 6 nucléotides en longueur.

22. Composition pour l'utilisation selon l'une quelconque des revendications 15 à 21, où le polynucléotide immunostimulatoire comprend la séquence 5'-purine-purine-C-G-pyrimidine-pyrimidine-3'.

23. Composition pour l'utilisation selon l'une quelconque des revendications 15 à 21, où le polynucléotide immunostimulatoire comprend la séquence 5'-pyrimidine-pyrimidine-C-G-purine-purine-3'.

24. Composition pour l'utilisation selon l'une quelconque des revendications 15 à 21, où le polynucléotide immunostimulatoire comprend la séquence (TCG)ₙ, où n = 1 à 3.

25. Composition pour l'utilisation selon l'une quelconque des revendications 15 à 21, où le polynucléotide immunostimulatoire comprend une séquence sélectionnée dans le groupe consistant en GCGCGC, AGCGCT, CGATCG, CGCGCG, ACCGGT, GACGATCGTC, CGACGATCGTCG, CGACGACGATCGTCGTCG, CAACGTTG, AACAACGTTGTT, CAACAACGGTTGTTG, GACGTC, ATCGAT, CCTACG, TCGCGA, ACGT, ACGATCGT, ACAACGTTGT et AACGTT.

26. Composition pour l'utilisation selon l'une quelconque des revendications 15 à 25, où le mammifère est un être humain.

27. Composition pour l'utilisation selon l'une quelconque des revendications 15 à 26, où le polynucléotide immunostimulatoire est dans un plasmide.

28. Composition pour l'utilisation selon l'une quelconque des revendications 15 à 26, où le polynucléotide immunostimulatoire est dans un cosmide.


### SEQUENCE LISTING

(1) GENERAL INFORMATION:
   (i) APPLICANT: Carson, Dennis A. Raz, Eyal
   (ii) TITLE OF INVENTION: RECOMBINANT GENE EXPRESSION VECTORS AND METHODS FOR USE OF SAME TO ENHANCE THE IMMUNE RESPONSE OF A HOST TO AN ANTIGEN
   (iii) NUMBER OF SEQUENCES: 20
   (iv) CORRESPONDENCE ADDRESS:
      (A) ADDRESSEE: Fish Richardson P.C.
      (B) STREET: 4225 Executive Square, Suite 1400
      (C) CITY: La Jolla
      (D) STATE: California
      (E) COUNTRY: USA
      (F) ZIP: 92037
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30B
   (vi) CURRENT APPLICATION DATA:
      (A) APPLICATION NUMBER: US
      (B) FILING DATE:
      (C) CLASSIFICATION:
   (viii) ATTORNEY/AGENT INFORMATION:
      (A) NAME: Howells, Stacy L.
      (B) REGISTRATION NUMBER: 34,842
      (C) REFERENCE/DOCKET NUMBER: 07340/044001
   (ix) TELECOMMUNICATION INFORMATION:
      (A) TELEPHONE: (619) 678-5070
      (B) TELEFAX: (619) 678-5099
(2) INFORMATION FOR SEQ ID NO:1:
   (i) SEQUENCE CHARACTERISTICS:
      (A) LENGTH: 6 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:1:
      AACGTT 6
(2) INFORMATION FOR SEQ ID NO:2:
   (i) SEQUENCE CHARACTERISTICS:
      (A) LENGTH: 6 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:2:
      TTGCAA 6
(2) INFORMATION FOR SEQ ID NO:3:
   (i) SEQUENCE CHARACTERISTICS:
      (A) LENGTH: 6 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..6
   (xi) SEQUENCE DESCRIPTION: SEQ ID NO:3:
      GCGCGC 6
(2) INFORMATION FOR SEQ ID NO:4:
   (i) SEQUENCE CHARACTERISTICS:
      (A) LENGTH: 6 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..6
   (xi) SEQUENCE DESCRIPTION: SEQ ID NO:4:
      GACGTC 6
(2) INFORMATION FOR SEQ ID NO:5:
   (i) SEQUENCE CHARACTERISTICS:
      (A) LENGTH: 6 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..6
   (xi) SEQUENCE DESCRIPTION: SEQ ID NO:5:
      AGCGCT 6
(2) INFORMATION FOR SEQ ID NO:6:
   (i) SEQUENCE CHARACTERISTICS:
      (A) LENGTH: 6 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..6
   (xi) SEQUENCE DESCRIPTION: SEQ ID NO:6:
      ATCGAT 6
(2) INFORMATION FOR SEQ ID NO:7:
   (i) SEQUENCE CHARACTERISTICS:
      (A) LENGTH: 6 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..6
   (xi) SEQUENCE DESCRIPTION: SEQ ID NO:7:
      CGATCG 6
(2) INFORMATION FOR SEQ ID NO:8:
   (i) SEQUENCE CHARACTERISTICS:
      (A) LENGTH: 6 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..6
   (xi) SEQUENCE DESCRIPTION: SEQ ID NO:8:
      CGTACG 6
(2) INFORMATION FOR SEQ ID NO:9:
   (i) SEQUENCE CHARACTERISTICS:
      (A) LENGTH: 6 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..6
   (xi) SEQUENCE DESCRIPTION: SEQ ID NO:9:
      CGCGCG 6
(2) INFORMATION FOR SEQ ID NO:10:
   (i) SEQUENCE CHARACTERISTICS:
      (A) LENGTH: 6 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..6
   (xi) SEQUENCE DESCRIPTION: SEQ ID NO:10:
      TCGCGA 6
(2) INFORMATION FOR SEQ ID NO:11:
   (i) SEQUENCE CHARACTERISTICS:
      (A) LENGTH: 6 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1
   (xi) SEQUENCE DESCRIPTION: SEQ ID NO:11:
      ACCGGT 6
(2) INFORMATION FOR SEQ ID NO:12:
   (i) SEQUENCE CHARACTERISTICS:
      (A) LENGTH: 4 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..4
   (xi) SEQUENCE DESCRIPTION: SEQ ID NO:12:
      ACGT 4
(2) INFORMATION FOR SEQ ID NO:13:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..10
   (xi) SEQUENCE DESCRIPTION: SEQ ID NO:13:
      GACGATCGTC 10
(2) INFORMATION FOR SEQ ID NO:14:
   (i) SEQUENCE CHARACTERISTICS:
      (A) LENGTH: 8 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..8
   (xi) SEQUENCE DESCRIPTION: SEQ ID NO:14:
      ACGATCGT 8
(2) INFORMATION FOR SEQ ID NO:15:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..12
   (xi) SEQUENCE DESCRIPTION: SEQ ID NO:15:
      CGACGATCGT CG 12
(2) INFORMATION FOR SEQ ID NO:16:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..18
   (xi) SEQUENCE DESCRIPTION: SEQ ID NO:16:
      CGACGACGAT CGTCGTCG 18
(2) INFORMATION FOR SEQ ID NO:17:
   (i) SEQUENCE CHARACTERISTICS:
      (A) LENGTH: 8 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..8
   (xi) SEQUENCE DESCRIPTION: SEQ ID NO:17:
      CAACGTTG 8
(2) INFORMATION FOR SEQ ID NO:18:
   (i) SEQUENCE CHARACTERISTICS:
      (A) LENGTH: 10 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..10
   (xi) SEQUENCE DESCRIPTION: SEQ ID NO:18:
      ACAACGTTGT 10
(2) INFORMATION FOR SEQ ID NO:19:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..12
   (xi) SEQUENCE DESCRIPTION: SEQ ID NO:19:
      AACAACGTTG TT 12
(2) INFORMATION FOR SEQ ID NO:20:
   (i) SEQUENCE CHARACTERISTICS:
      (A) LENGTH: 14 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: CDS
      (B) LOCATION: 1..14
   (xi) SEQUENCE DESCRIPTION: SEQ ID NO:20:
      CAACAACGTT GTTG 14